# EUROPEAN PATENT APPLICATION

(11) **EP 1 961 394 A2**
(43) Date of publication of application: **27.08.2008**
(21) Application number: 08101856.6
(22) Date of filing: 21.02.2008
(51) Int. Cl.: A61B 18/14, A61N 1/05, A61M 5/142

(54) **Combination electrical stimulating and infusion medical device and method**

(30) Priority: 23.02.2007 US 678516; 29.06.2007 US 771757
(71) Applicant: Vilims, Bradley D., Evergreen CO 80439 (US); Woolfson, Steven B., Boston, MA 02205 (US); Rittman, William J., III, Lynnfield, MA 01940 (US)
(72) Inventor: Vilims, Bradley D., Evergreen CO 80439 (US); Woolfson, Steven B., Boston, MA 02205 (US); Rittman, William J., III, Lynnfield, MA 01940 (US)
(74) Representative: Harrison Goddard Foote

(57) **Abstract**

A combined electrical and chemical stimulation lead is especially adapted for providing treatment to the spine and nervous system. The stimulation lead includes electrodes that may be selectively positioned along various portions of the stimulation lead in order to precisely direct electrical energy to ablate or electrically stimulate the target tissue. Embodiments of the stimulation lead include single or multiple lead elements. The multiple lead element embodiments can be selectively deployed to cover a targeted area. The lead may also includes central infusion passageway(s) or lumen(s) that communicates with various infusion ports spaced at selected locations along the lead to thereby direct the infusion of nutrients/chemicals to the target tissue. Some embodiments utilize a disposable sheath in combination with a reusable stimulation lead.

## Description

### CROSS REFERENCE TO RELATED APPLICATION

This application is a continuation-in-part of co-pending application Serial No. 11/678,516 filed on February 23, 2007 entitled "COMBINATION ELECTRICAL STIMULATING AND INFUSION MEDICAL DEVICE", which is a continuation-in-part of co-pending Application Serial No. 11/107,553, filed on April 14, 2005, entitled "COMBINATION ELECTRICAL STIMULATING AND INFUSION MEDICAL DEVICE", which is a continuation-in-part of co-pending application of application serial no. 11/033,591, filed on January 11, 2005, entitled "COMBINATION ELECTRICAL STIMULATING AND INFUSION MEDICAL DEVICE", the disclosures of which are hereby incorporated by reference herein.

### TECHNICAL FIELD OF THE INVENTION

The present invention relates generally to electrical stimulation leads and chemical infusion catheters for treatment of medical conditions, and more particularly, to a system, method and device for providing combined electrical stimulation and chemical/drug infusion for treatment of targeted tissue such as intervertebral discs, SI joints, various vertebral structures, and various nerve groups along the spine to include the spinal cord.

### BACKGROUND OF THE INVENTION

It is known that immersing certain cell types within an electrical field will cause these cells to proliferate thus facilitating tissue repair. One known use of an electrical field for such repair is "in bone" stimulators that are implanted in fractures and/or spinal fusions. Another type of treatment has recently been developed for spinal conditions wherein target tissue is stimulated by an electrical lead using radio-frequency energy to induce a thermal lesion in the target tissue. In this type of procedure, the therapeutic benefit is intended to derive from heating the target tissue and not from immersing the tissue in an electric field. Thus, the electrical lead in this treatment is strictly for use in heating the tissue, and there is no therapeutic electrical field generated. Chemical treatment of target tissues has also been developed by use of various types of infusion catheters.

For both electrical and thermal stimulation, an electrical current generator, commonly referred to as a pulse generator, may be used to transmit a pulse of electrical current to an implanted stimulation lead that has been precisely placed to transmit the electrical or thermal energy from the electrodes to the target tissue in order to treat the particular condition. For chemical stimulation, one or more drugs or nutrients are delivered by a pump that transfers a desired quantity and frequency of the drug/nutrient through an infusion port of the catheter to the target tissue. For chemical stimulation as well as electrical/thermal stimulation, implanted pumps and generators can be used to deliver the electrical and chemical stimulation as opposed to trans-dermal delivery devices. More particularly, implanted pulse generators (IPG) as well as implanted drug dispensers (IDP) are commonly used so that patients do not have to return to a medical facility each time treatment is to be conducted.

The intervertebral disc (IVD) provides separation, shock absorption, and controlled motion between vertebral bodies. The disc is comprised of a central nucleus of a semi-fluid mass of mucoid material, (nucleus pulposus), an outer more dense collagen ring (annulus fibrosis), and a thin, metabolically active cellular layer separating the nucleus and the outer collagen ring, referred to as the annular nuclear interface/transitional zone. Disc nutrition is tenuous at best and is provided by diffusion through the vertebral end plate in contact with the outer surface of the disc. As a result, a disc has limited ability to heal or regenerate. Due to age, injury or other conditions, cracks or fissures may develop in the wall of invertebral discs causing a chronic source of pain in many patients. Additionally, the inner disc tissue (nucleus) will frequently cause the disc to bulge or herniate into the fissures in the outer region of the disc, thus causing nerve tissue therein to generate pain signals.

Current treatment for such disc disorders include analgesics, physical therapy and epidural steroid injections. Success with these treatments is frequently disappointing and the patient will all too often have to undergo spinal fusion. Spinal fusion is a very invasive, biomechanically altering, and marginally effective treatment.

One relatively new procedure has been developed to treat such disc ailments and general discogenic back pain. As an alternative to other surgical procedures for patients who suffer from back pain caused by certain types of disc disorders, this new procedure is made possible by use of thermal stimulation leads that provide precise temperature control in the delivery of thermal energy to target tissue. This procedure, commonly referred to as intradiscal electro-thermal annuloplasty (IDET) was initially believed to function by cauterizing nerve endings within the disc wall to assist in reduction of pain, and the heat produced by the stimulation leads would also thicken the collagen of the disc wall thereby promoting healing of the damaged disc. IDET has proven in some cases to be a minimally invasive procedure to treat these types of disc ailments. However, recent research, and clinical experience has cast doubt as to the exact method of action. More specifically, for percutaneous treatments like IDET, the general operating premise in these procedures, is to heat, either through conduction or induction, causing collagen restructuring and nociceptor coagulation within the disc that would stabilize the structure, and dennervate the painful discs while retaining the motion segment and thus reduce the need for fusion. While these procedures have proven more effective than placebo, the results are far from acceptable. Research has demonstrated that collagen modulation and nociceptor coagulation is unlikely to be the mechanism of action, and that these devices may simply create injury patterns, that in a small subset of patients, stimulates a regenerative response, thereby accounting for the better than placebo results.

Combination electrical stimulators and chemical infusion catheters are known for purposes of treating various spine and brain ailments. One reference that discloses such a combination device is the invention in U.S. Publication No. US2004/0243206. This reference specifically discloses a combination electrical and stimulation lead for stimulation of a person's nerve tissue in the brain. One or more electrodes are located along the lead body and are adapted to be positioned proximate the target nerve tissue and to deliver electrical stimulation pulses transmitted through the lead to the target nerve tissue. One or more infusion ports located along the lead body are adapted for placement proximate the target nerve tissue and to deliver chemical stimulation pulses transmitted through the lead to the target nerve tissue.

While combination electrical and stimulation leads may be known, special considerations must be made for use of such devices for intervertebral disc treatment.

Placement of a stimulation lead within a disc can be quite difficult. Because a disc does not have a uniform density, known stimulation leads can be quite difficult to place and may require the attending physician to make multiple attempts for proper placement or abandon the procedure. Of course, multiple placement attempts greatly increase the invasive nature of the procedure and therefore create unnecessary tissue damage and increased risk. Inability to perform the procedure denies the patient a therapeutic option. Improper placement of the stimulation lead can also result in the undesirable damage of nerve tissue that is not contributing to the chronic pain or other ailments. Because of the overall metabolically inactive nature of the disc, it is also important that chemical infusion be precisely targeted to contact the damaged area of the disc with the delivered chemicals/nutrients, otherwise inaccurate delivery to non-damaged portions of the disc can reduce the effectiveness of the procedure. Thus, there is a need for a combination electrical and chemical stimulation lead that can be precisely placed with a high rate of success on a first attempt.

The IVD is also a motion segment of the body that is subjected to many flexion/extension/rotation cycles every day. In some procedures, it may be necessary to keep the stimulation lead emplaced for long periods of time, such as weeks or perhaps months. Thus, it is desirable to have a stimulation lead that maintains a small profile, yet is resilient enough to withstand the risk of permanent deformation or shearing during treatment and removal of the stimulation lead after treatment.

Many complaints of lower back and leg pain have been attributed to herniated disk related injuries to the spinal column. Extensive therapy and treatment is often unsuccessful in alleviating such pain since some of these problems are actually associated with symptomatic sacroiliac dysfunction or instability. Other terms to describe sacroiliac ailments include sacroiliac joint complex, sacroiliac joint dysfunction, and others. One reference that discloses the use of a bone implant to provide stability and compression for immobilization of the SI joint is the US Pat. No. 6,053,916. One reference that discloses methods for treatment of pain caused by an SI joint dysfunction includes US Pat. App. Publication No. US 2006/0217705. This reference discloses a number of electro-surgical devices in which energy is directed to a targeted region of tissue. A probe is inserted into the target site within the sacroiliac region of the patient's body and energy is delivered to the probe. At the location of the probe, the tissue is ablated thereby creating lesions. In the case of contact of the probe with neural tissues, denervation is achieved which therefore can reduce or eliminate pain associated with the particular dysfunction being treated.

With respect to neural ablation to alleviate symptomatic pain associated with numerous types of spine ailments, current stimulation leads are limited in the provision of ablative heat based on the size of the electrodes, their spacing along the lead, and their particular positioning relative to the targeted nerve group. In many instances, it may be necessary to move the stimulation lead during a procedure to cover all of the targeted tissue and repeatedly apply electrical energy to the lead. In other circumstances, it may be necessary for the introducer needle to be completely removed and reinserted in an adjacent position to then reposition the stimulation lead in order to cover the targeted tissue. Multiple lead position changes during a procedure of course increases the invasive nature of the procedure and also introduces additional risk that multiple needle insertions will damage non-targeted tissue.

While the prior art may disclose various devices and methods for treatment of targeted tissue throughout the body, there is still a need for improved devices and methods for treatment, to include devices and methods wherein electrical stimulation as well as chemical infusion may be provided with the same stimulation device. Additionally, there is a need for an electrical stimulation device that has the capability to provide various types of electrical stimulation and ablative patterns thereby increasing the chances that a procedure will be successful since the patterns can be selected to cover targeted tissue based on the condition of the particular patient and the ailment to be treated.

### SUMMARY OF THE INVENTION

In accordance with the present invention, a combined electrical and chemical stimulation device is provided that is especially adapted for treatment of various types of ailments associated with the spine and nervous system.

With respect to treatment of an intervertebral disc, the stimulation device is in the form of a stimulation lead designed to be placed in the disc percutaneously through an introducer needle using an extra-pedicular approach; however, micro-surgical or opensurgical techniques may also be utilized. More specifically, the device of the present invention is specifically designed to facilitate placement proximate to the metabolically active cellular, nuclear, annular interface layer by use of one or more selected embodiments including a straight, curved or bent tip, as well as a variable stiffness tip. Selection of one of these embodiments allows the physician to precisely navigate the lead through the nucleus of the disc. In yet another embodiment of the present invention, the stimulation lead may be placed directly into the nuclear annular interface by use of an introducer needle having a bent tip, and use of a stimulation lead having a straight tip that can take a substantially linear path to reach the target tissue.

With respect to treatment of an SI joint, the same type of stimulation device used for treating the intervertebral disc can be used. Generally, the procedure for treatment of the SI joint involves first the placement of an introducer needle along the curvature of the sacrum with the needle tip ultimately advanced to the superior edge of the sacrum lateral to the sacral foramen and medial to the SI joint. The stimulation lead may be then placed through the introducer needle and advanced to the tip of the introducer needle. The introducer needle is then withdrawn along a specified length of the stimulation lead to expose the active number of contacts necessary to denervate the targeted sacral nerve lateral branches.

The structure of the stimulation lead of the present invention in some embodiments is characterized by an elongate and tubular shaped body including one or more electrodes located along selected portions of the lead body and adapted for positioning proximate the target tissue to deliver electrical stimulation pulses transmitted through the lead. In some embodiments, the electrodes extend circumferentially around a selected length or portion of the lead since it is difficult to orient a specific lateral side of the lead against target tissue. One or more infusion ports may also be located along the lead body and are adapted to be positioned proximate the target tissue to deliver selected chemicals/nutrients. In other embodiments, one large continuous electrode may cover the entire distal portion of the stimulation lead, and this type of lead is especially adapted for ablation procedures.

In some embodiments of the present invention, instead of a single tubular shaped body, the stimulation lead may have a plurality of lead elements with a common base, and the stimulation elements may be selectively deployed at the targeted tissue site. The separate stimulation elements may be deployed by a number of deployment mechanisms to include spring elements, hydraulic force, and selected materials with elastomeric and resilient characteristics that expand the lead elements in the desired configuration once it is freed from within an introducer needle or sheath. In yet other embodiments of the present invention, the stimulation elements may be flat or planar as opposed to tubular shaped. In some of the embodiments, a central stylet can be used to help guide the stimulation lead and to provide some additional rigidity to prevent inadvertent buckling or displacement of the lead.

Once the stimulation lead is correctly positioned, the lead is then connected to a pulse generator for delivery of electrical energy to the electrodes located on the distal portion of the stimulation lead. The electrical circuit can be completed by either use of a grounding pad placed on the patient or by the stimulation lead itself where the electrodes are provided in various combinations of anodes and cathodes.. For those embodiments that include infusion ports, the lead may also be connected to an infusion pump that provides a controlled delivery of chemicals/nutrients through the lead to the target tissue. Preferably, the electrical pulse generator and infusion pump are implanted medical devices. These pulse generator and infusion pump devices are also preferably rechargeable and refillable. Another generally desirable characteristic of pulse generators includes those having a capability to produce either constant or variable current. It is also desirable to provide electrical contacts/electrodes that are linked in series, parallel, or combinations thereof which allow selective activation of all or a selected group of the electrodes. Other desirable general characteristics for an infusion pump are those pumps which (i) control infusion material at either a constant or variable rate, and at a constant or variable pressure, or constant or variable volume, (ii) provide automatic compensation for varying infusion pressures, and (iii) have anti-back flow capability to prevent backflow of infusion material through the stimulation lead, as well as pressure safety valves to compensate for overpressure situations. Furthermore, the pump, pulse generator and stimulation lead may be coated with an antibacterial coating to decrease the risk of infection. The pulse generator and pump may also incorporate appropriate alarms to notify of infusion and stimulation failure/abnormality.

Particular embodiments of the present invention provide one or more advantages in terms of navigation of the stimulation lead, as well as placement of the infusion ports and electrodes for effectively delivering electrical and chemical treatment. More specifically, the particular shape of the stimulation lead, as well as the particular placement of the electrodes and infusion ports are especially adapted for delivering the electrical stimulation and chemical infusion to target tissue. A stiffening or support element may be incorporated in the wall of the stimulation lead to ensure the lead does not prematurely shear or otherwise structurally fail during use and removal. The stiffening element is preferably in the form of an elongate support that extends longitudinally within the wall of the stimulation lead and terminating near the distal tip of the lead.

Other embodiments of the present invention provide advantages by use of a disposable sheath that can be used in combination with a reusable stimulation lead. The disposable sheath has electrical contacts forming the electrodes of the device when used. The reusable stimulation lead is placed inside the disposable sheath wherein the electrodes of the stimulation lead make electrical contact with the electrodes of the disposable sheath. Temperature sensing elements may be incorporated in the stimulation lead, such as thermocouples or RTDs in order to measure temperature at the active electrical areas to ensure uniform lesioning, and to otherwise provide additional safety to the procedure such that excessive energy is not applied. Since the disposable sheath is in thermal/electrical contact with the inner reusable stimulation lead, accurate temperature sensing can still take place when the temperature sensing elements are incorporated on the stimulation lead. It is also contemplated that temperature-sensing elements may be incorporated on the disposable sheath wherein the bundle of conductors from the temperature sensing elements are separately routed through the sheath.

Use of the disposable sheath allows great flexibility in determining the pattern, size, and general configuration of a stimulation lead to be used in many types of different medical procedures. By providing a reusable stimulation lead, the cost in conducting a procedure is reduced since only the disposable sheath is disposed of after use and not the entire assembly.

In yet another embodiment of the invention, an inflatable member may be used with a stimulation lead such that the inflatable member can shift or adjust the exact positioning of the stimulation lead to more accurately apply the electrical or thermal energy to the targeted area.

Further advantages and features of the present invention will become apparent from a review of the following detailed description, taken in conjunction with the drawings.

### BRIEF DESCRIPTION OF THE DRAWINGS

Reference is now made to the following detailed description taken in conjunction with the accompanying drawings in order for a more thorough understanding of the present invention.
Figure 1 illustrates the present invention including a stimulation lead inserted in an intervertebral disc, and a stimulation source that provides a controlled delivery of electrical field energy and chemicals/nutrients through the stimulation lead;
Figure 2 is a greatly enlarged cross-section of the working distal portion of one preferred embodiment of the stimulation lead of the present invention;
Figures 3-7 are greatly enlarged side or elevation views illustrating other preferred embodiments of the stimulation lead;
Figure 8 is a greatly enlarged cross-section of the working distal portion of another preferred embodiment that incorporates a stiffening element;
Figure 9 is a section taken along line 9-9 of Figure 8;
Figure 10 illustrates another preferred embodiment of the present invention in the form of an introducer needle having a bent distal end for placement of the stimulation lead directly into the nuclear annular interface of an intervertebral disc;
Figures 11-13 illustrate further embodiments of the present invention wherein the electrodes and infusion ports are dispersed substantially along the entire length of the stimulation lead;
Figure 14 illustrates a cross section of a further embodiment of the present invention wherein a dual lumen is provided enabling greater selective control of infusion through designated portions of the stimulation lead;
Figure 15 illustrates yet a further embodiment of the present invention wherein an inflatable member is provided near the distal end of the stimulation lead to help anchor the lead after emplacement;
Figure 16 illustrates the embodiment of Figure 15 but the inflatable member being provided near the proximal end of the stimulation lead.
Figure 17 illustrates the stimulation lead of Figure 14 inserted in an invertebral disc wherein the lead can selectively treat two targeted treatment zones or areas within the disc;
Figure 18 illustrates a cross section of yet a further embodiment of the present invention wherein the body of the stimulation lead is made from a dissolvable matrix with the stimulating electrical contacts embedded therein;
Figure 19 illustrates a cross section of a further variation of the embodiment of Figure 18 wherein the stimulation lead has a preconfigured bend at the distal end thereof and no central lumen;
Figure 20 is a cross-section taken along line 20-20 of Figure 18, and further illustrating the use of an outer membrane which may help maintain the integrity of the stimulation lead when emplaced, as well as to control the rate at which the constituents incorporated within the dissolvable matrix are allowed to diffuse into the intervertebral disc; and
Figure 21 is a posterior/dorsal view of the sacroiliac region with an introducer needle being positioned for insertion along the SI joint;
Figure 22 is an enlarged posterior/dorsal view of the sacrum bone showing the introducer needle fully inserted;
Figure 23 is another enlarged anterior view of the sacrum bone showing the introducer needle withdrawn a selected length thereby exposing a specific number or group of electrical contacts/electrodes for denervation of selected sacral nerves.
Figure 24 illustrates yet another preferred embodiment of the present invention showing a stimulation lead having a substantially uniform curvature along the length of the stimulation lead along with a bent distal tip;
Figure 25 illustrates the embodiment of Fig. 24 wherein the stimulation lead is inserted adjacent a selected vertebral structure, for conducting treatment such as ablation of a ventral nerve group;
Figure 26 illustrates yet another preferred embodiment of the present invention wherein the stimulation lead is substantially liner or straight, and a central needle or stylet is passed through a central lumen of the stimulation lead;
Figure 27 illustrates yet another preferred embodiment wherein the stimulation lead has a pair of deployable stimulation elements;
Figure 28 illustrates the embodiment of Fig. 27 and showing the stimulation elements deployed;
Figure 29 illustrates the embodiment shown in Figs. 27 and 28 but further including a central stylet to assist the guidance of the stimulation lead;
Figure 30 illustrates yet another preferred embodiment of the present invention wherein the pair of stimulation elements are more closely spaced, and are substantially parallel to one another;
Figure 31 is a fragmentary perspective view of a portion of a spine and the stimulation lead of Fig. 30 for purposes of conducting a procedure for stimulation of the spinal cord;
Figure 32 is a lateral or side view of the stimulation lead of Fig. 31 for the procedure shown in Fig. 31;
Figure 33 illustrates yet another preferred embodiment of the present invention having three stimulation elements arranged in a planer or side-by-side fashion.
Figure 34 is an end view of stimulation lead of Fig. 33 illustrating the planer or side-by-side arrangement of the stimulation elements.
Figure 35 illustrates yet another embodiment similar to the embodiment of Fig. 33, but the stimulation elements are deployed by the elastomeric force of the stimulation lead material and a different pattern of electrodes and infusion ports are provided;
Figure 36 illustrates the manner in which the embodiments of Figs. 33 and 35 may be stored within the introducer needle prior to deployment;
Figure 37 is an end view of Fig. 36 illustrating the stimulation lead within the introducer needle prior to deployment;
Figure 38 illustrates yet another embodiment of the present invention wherein the stimulation lead includes three stimulation elements spaced from one another in a triangular configuration;
Figure 39 is an end view of the embodiment of Fig. 38 illustrating the particular arrangement of the stimulation elements;
Figure 40 illustrates yet another embodiment of the present invention comprising a pair of stimulation elements that are deployed by a deploying tether;
Figure 41 illustrates the embodiment of Fig. 40 showing the stimulation elements deployed by retraction of the deploying tether;
Figure 42 is an end view of Figure 41;
Figure 43 is a perspective view of the embodiment of Figure 40, also illustrating the stimulation elements deployed;
Figure 44 illustrates yet another preferred embodiment of the present invention comprising a pair of deployable stimulation elements deployed by a movable sheath;
Figure 45 illustrates the embodiment of Fig. 44 showing the stimulation elements partially deployed;
Figure 46 is a perspective view of the embodiment of Fig. 44 showing the stimulation elements fully deployed;
Figure 47 is a cross sectional view of yet another embodiment of the present invention wherein hydraulic pressure is used to deploy the stimulation elements by fluid which is pumped into a central lumen of the stimulation lead;
Figure 48 illustrates yet another embodiment of the present invention wherein the stimulation lead is substantially flat or planer, and the stimulation elements include continuous electrodes that extend along the length of the elements;
Figure 49 illustrates yet another embodiment of the present invention wherein the stimulation lead is inflatable;
Figure 50 illustrates the embodiment of Figure 49 wherein the stimulation lead has been inflated;
Figure 51 illustrates another stimulation lead similar to the one shown in Figure 30, but further including a separate inflatable member that is secured to the stimulation lead for fine positioning of the stimulation lead;
Figure 52 is a side view of the stimulation lead of Figure 51;
Figure 53 is a fragmentary perspective view of a portion of a spine and the stimulation lead of Figure 51 for purposes of conducting a procedure for stimulation of the spinal cord;
Figure 54 illustrates yet another preferred embodiment of the present invention having three stimulation elements arranged in a triangular configuration, along with a central infusion tube;
Figure 55 is an end view taken along line 55-55 of Figure 54;
Figure 56 illustrates yet another preferred embodiment of the present invention utilizing coils of wire as the electrode elements;
Figure 57 is a cross-section of Figure 56 illustrating the stimulation lead in use along with a steering element such as a stylet to adjust the shape of the stimulation lead;
Figure 58 illustrates yet another preferred embodiment of the present invention in the form of a stimulation lead that can be either disposable, or reusable when used with a disposal outer sheath;
Figure 59 is an enlarged fragmentary perspective view of the body of the stimulation lead of Figure 58 illustrating details thereof;
Figure 60 is an exploded perspective view of a disposable outer sheath usable with a stimulation lead;
Figure 61 is an assembled perspective view of the disposable sheath of Figure 60;
Figure 62 is a perspective view of the stimulation lead similar to the stimulation lead of Figure 58, however, the stimulation lead of Figure 62 being especially adapted as a reusable stimulation lead with no infusion capability;
Figure 63 is an enlarged fragmentary perspective cross-section of the sheath of Figure 61 and the body of the stimulation lead of Figure 62 illustrating how the lead is inserted within the sheath;
Figure 64 illustrates a cross-section of yet another reusable stimulation lead especially adapted for use with a disposable sheath;
Figure 65 illustrates a component of another type of reusable sheath along with a stiffening opturator;
Figure 66 illustrates an enlarged exploded perspective view of various components of the disposable sheath and reusable stimulation leads of Figures 64 and 65, particularly showing construction details and the manner in which electrical connection is achieved between the electrodes of the outer sheath and stimulation lead;
Figure 67 is a cross-section of the assembled disposable sheath of Figure 66 showing the reusable stimulation lead of Figure 64 used with the sheath; and
Figure 68 is another enlarged exploded perspective view of an alternate shaped bracket used to electrically interconnect the sheath to the stimulation lead.

### DETAILED DESCRIPTION

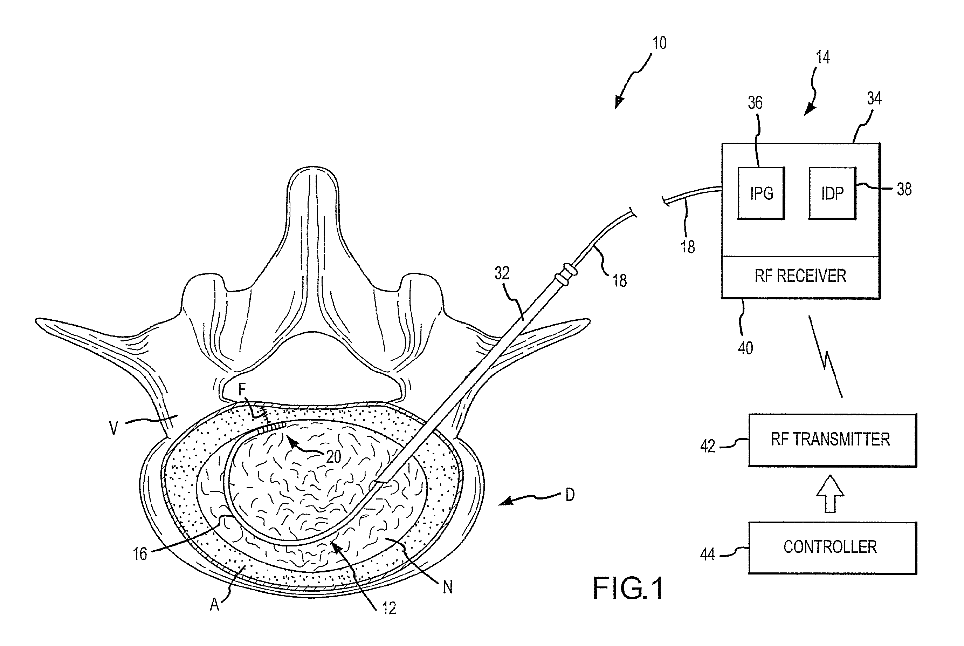

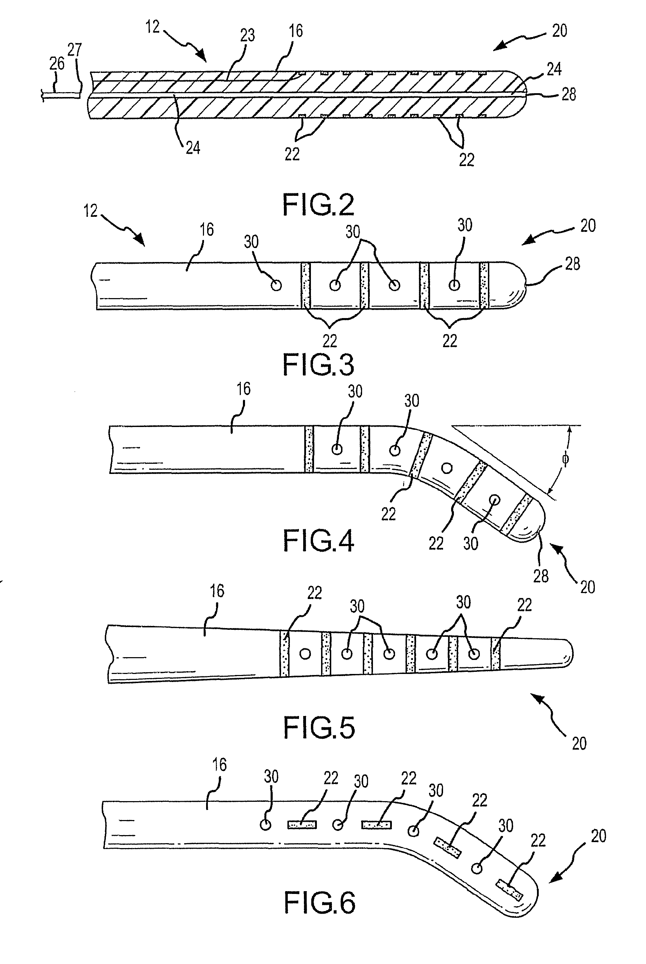

Referring to Figures 1 and 2, the system 10 of the present invention is shown that includes a combination electrical and chemical stimulation device 12, a stimulation source 14 that communicates with the stimulation device 12 for delivering electrical energy and chemicals to the stimulation device, and an interventional device such as an introducer needle 32 that allows introduction of the stimulation lead. The stimulation device 12 is shown as inserted within an intervertebral disc D. The combination device 12 more particularly includes a percutaneous electrical and chemical stimulation lead 16 in the form of an elongate tubular member having a desired length and diameter allowing the lead 16 to be placed within the intervertebral disc of the patient to be treated. The working distal portion 20 of the stimulation lead 16 provides the desired stimulation through a plurality of electrodes 22 which are selectively positioned on the distal portion 20, along with a plurality of infusion ports 30 which allow delivery of chemicals/nutrients to target tissue. The proximal portion of the stimulation device 12 can be referred to as a lead extension 18 that connects to the stimulation source 14. The lead extension 18 can be made of the same type and diameter material as the stimulation lead 16, or may be made of a different type of material and diameter.

Referring specifically to Figure 2, in a first embodiment of the stimulation lead, a plurality of circumferentially extending electrodes 22 are positioned at the distal portion 20. The electrodes 22 are also spaced longitudinally along the distal portion 20. The electrodes produce an array of electrical field energy, and the target tissue is immersed in the electrical field. One or more electrical conductors 23 extend through the interior of the stimulation lead 16 in order to transmit the electrical impulses to the electrodes 22. It is preferable to utilize a single conductor 23 along the major length of the lead, and then provide branch conductors (not shown) at the distal portion 20 that then extend to contact the various electrodes. The branch conductors could be a linearly arranged set of wire extensions extending between each electrode, or any other advantageous combination of wire conductors to interconnect the electrodes. Use of a single conductor is a more robust design as opposed to multiple smaller conductors that are more prone to breakage as a result of the motion cycles of the IVD. It is also contemplated that the electrode could be a single electrode wound in a helical pattern about the distal portion 20. Thus in this helical pattern, only one conductor 23 would be required with no additional branch conductors. In order to generate the desired intensity and size electrical field, the electrodes 22 can be disposed on the distal portion in a pattern or arrangement that best suits the electrical field to be generated. For example, in the helical pattern, the electrode could be wound with a tighter pattern to generate a more intense field, while a looser more spaced pattern would generate a less intense field. Of course, the particular signal or impulse current provided to the electrodes also determines the intensity of the field generated.

In order to provide chemical infusion, a central lumen or passageway 24 is formed through the stimulation lead. The central lumen 24 may extend completely through the lead thereby forming a distal opening 28 in the stimulation lead and providing one infusion port that is directed distally of the stimulation lead.

The stimulation lead 16 may be made of a homogeneous material, or may be made of differing materials that cause the stimulation lead to have either a more progressively stiff or more progressively flexible characteristic as the lead extends in the distal direction. Depending upon the manner in which the stimulation lead is to be emplaced, it may be desirable to use either the more progressively stiff or more progressively flexible arrangement.

In accordance with the method of the present invention, a stylet (not shown) is first inserted through the introducer needle 32. The introducer needle 32 is emplaced by penetrating the skin and muscle tissue, and ultimately into the disc D. When the introducer needle has penetrated the disc, the stylet is removed and the stimulation lead 16 is then inserted through the lumen of the introducer needle.

Referring again to Figure 1, the stimulation lead 16 is illustrated as being emplaced within the disc D. This disc D is shown in cross section along with an adjacent vertebra V. The stimulation lead 16 is shown as taking an arcuate or curved path through the disc nucleus N in order to be precisely positioned at the area of the disc to be treated, illustrated as a fissure F which has developed adjacent the spinal fluid sac (not shown). The other primary features of the disk D are also illustrated including the annulus fibrosis A and the thin layer L defining the annular nuclear interface/transitional zone.

The stimulation source 14 is preferably an implantable medical device 34 including both an IPG (implantable pulse generator) 36 and an IDP (implantable drug dispenser) 38. The implantable device 34 could be contained within a single structural housing, or two separate housings, one for the IPG 36, and one for the IDP 38. The IPG and IDP can both be self-contained devices with internal control for preset delivery of electrical and chemical pulses. Alternatively, an external controller 44 could be used to modify the desired treatment protocol by use of RF transmission wherein an implantable RF receiver 40 is integrated with the IPG 36 and IDP 38. The RF receiver 40 could also be housed within the same implantable medical device 34, or could be a separate implanted device. An external RF transmitter 42 transmits RF signals to control the delivery of electrical stimulation and chemicals to the stimulation lead 16. A controller 44 provides the specific instruction set for transmission by the RF transmitter 42.

In accordance with the apparatus and method of the present invention, there are a number of nutrients and medications that can be delivered by the stimulation lead. For nutrients, this list includes, but is not limited to, glucose, glucosamine, chondroitin, oxygen and oxygenating agents, anti-oxidants, anti-glycosylating agents, and pH buffers. For medications, these may include, without limitation, anti-inflammatory agents and growth factors, such as growth and differentiating factor-5 (GDF-5), transforming growth factor-beta (TGF-β), insulin-like growth factor-1 (IGF-1), and basic fibroblasts growth factor (bFGF). In terms of the types of electrical impulses provided to the electrodes 22, these electrical impulses may be continuous or variable over time, and may vary based upon voltage, amperage, and alternate current frequency.

Referring to Figure 3, a different arrangement is illustrated with respect to the location of the electrodes 22, and the single infusion port at distal opening 28 is supplemented with a plurality of additional infusion ports 30. In this embodiment, fewer electrodes are incorporated, yet additional infusion ports 30 are provided that are spaced longitudinally along the length of the lead 16 and placed between the electrodes 22.

Figure 4 shows another embodiment with a different arrangement of electrodes 22 and infusion ports 30 as well as a modification of the stimulation lead shape to include a bent distal tip having a chosen bend angle ⌀. The bend angle ⌀ helps define the path of travel of the lead within the disc nucleus during emplacement. In other words, imparting a particular bend angle on the distal tip of the stimulation lead causes the stimulation lead to travel in an arcuate path such as shown in Figure 1. Imparting a greater bend angle on the lead results in the stimulation lead traveling in a tighter arcuate path, while imparting a lesser bend angle generally results in the stimulation lead traveling in a broader arc or arcuate path.

Referring to Figure 5, another embodiment of the stimulation lead is illustrated wherein the lead has a progressively narrowing diameter towards the distal end thereof. With this type of stimulation lead, travel of the lead through the more dense annulus tissue is facilitated because the distal tip has a smaller frontal profile and is more easily controlled.

Referring to Figure 6, yet another embodiment of the stimulation lead is illustrated wherein the electrodes 22 are not formed circumferentially around the distal portion 20, but are formed more linearly along one side of the stimulation lead. Additionally, the infusion ports 30 may have more of an oval shape and be larger in size that facilitates greater volumetric infusion. This embodiment may be preferred when it is desired to more precisely direct the array of electrical energy to the target tissue. The electrical energy array that is created by circumferentially arranged electrodes result in transmission patterns having a radial or circular pattern extending away from the stimulation lead. Thus, a plurality of circumferentially arranged electrodes transmit energy in all directions to tissue that surrounds the stimulation lead. On the contrary, locating the electrodes only along one side or edge of the stimulation lead results in transmission of energy in a more linear and less radial pattern, and directed primarily orthogonal or perpendicular to the axis of the stimulation lead. The embodiment of Figure 6 also illustrates the distal end as being bent at a desired angle.

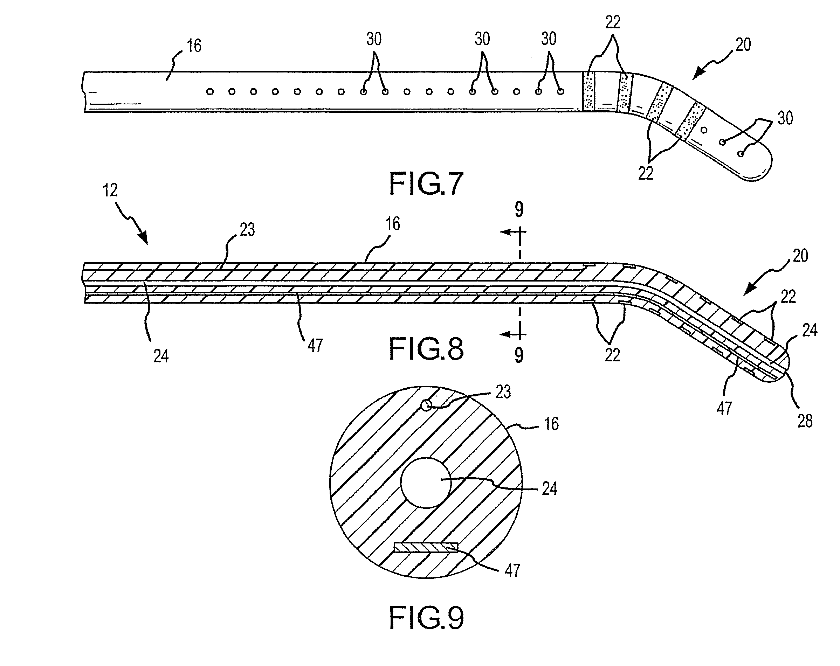

Figure 7 illustrates yet another embodiment of the stimulation lead wherein the electrodes 22 are concentrated at a particular location, and the infusion ports 30 are spaced in a pattern extending a greater longitudinal length of the lead. A stimulation lead in this particular arrangement may be particularly suitable for repair of a fissure located at a very defined position within the disc, yet if the disc shows great overall degeneration, it is preferable to provide nutrients to a greater length of the annulus whereby the infusion ports 30 can distribute nutrients to a greater length of the annulus.

Figure 8 illustrates yet another preferred embodiment of the present invention wherein a stiffening or strengthening member 47 is incorporated within the structural wall of the stimulation lead to provide increased strength to the lead without enlarging the frontal profile of the lead. As shown, the stiffening member 47 is an elongate member that extends longitudinally through the wall of the lead and terminates near the distal end thereof. The stiffening member is malleable to a degree that allows the lead to maintain some desired flexibility during emplacement, but increases the overall shear and torsional strength of the lead to prevent premature failure after emplacement or during removal. The member 47 could be made of a selected metal or thermoplastic approved for medical use.

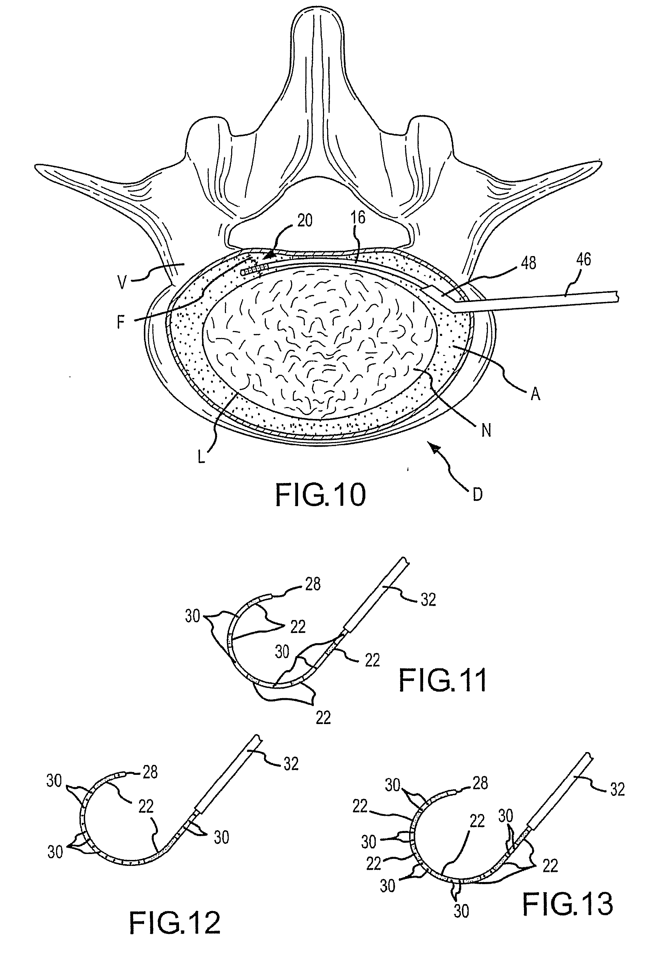

Referring to Figure 10, yet another embodiment of the invention is shown wherein an introducer needle 46 is not placed within the disc nucleus, but rather is placed only into the disc annulus, and then the stimulation lead 16 extends through the disc annulus to the target tissue, also shown as a fissure F. In this embodiment, it is preferable that the stimulation lead 16 exits the introducer needle through a bent distal portion 48 so that the lead travels in a more parallel fashion within the annulus and along a more linear path to the target tissue. In the event the distal opening 28 of the lead 16 is of a size which could allow nuclear tissue to clog or block the distal opening 28, a guide wire 26 (see Figure 12) may be inserted through the lumen 24 of the lead 16, and the distal tip 27 of the guide wire could be placed flush with the distal opening 28 in order to prevent clogging of the distal opening 28, as well as to provide additional rigidity for placement of the stimulation lead 16. If the guide wire 26 is used, then the guide wire 26 is removed prior to connecting the stimulation lead 16 to an IDP and/or IPG. Also, the central lumen may terminate before passing through the distal tip of the lead. Thus, all of the infusion ports 30 would be arranged on the lead to direct chemicals/nutrients in a perpendicular direction away from the axis of the lead.

Figures 11-13 illustrate yet further embodiments of the present invention wherein the electrodes 22 and infusion ports 30 are dispersed along substantially the entire length of the stimulation lead. In many cases, the disc to be treated has undergone such great degeneration that the entire disc is in need of treatment, as opposed to a more minor degenerative condition such as a single localized fissure. In such cases, it is advantageous to provide both electrical and chemical stimulation to as much of the disc as possible. The embodiments at Figures 11-13 show various combinations of the electrodes 22 and ports 30 that provide greater dispersion of the electrical and chemical stimulation. Specifically, the electrodes are larger and are spread out along a greater length of the lead. The infusion ports are also spread out along a greater length of the lead.

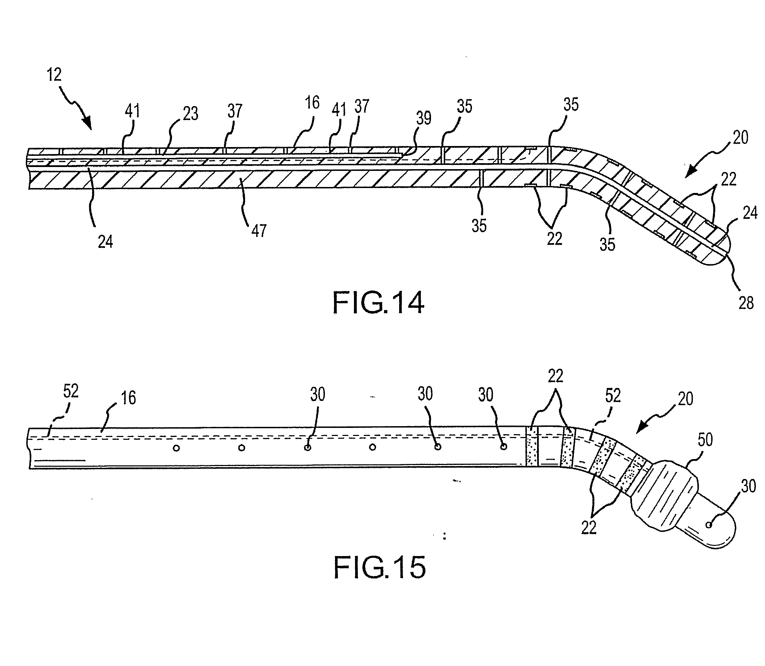

Figure 14 illustrates yet another embodiment of the invention wherein a second lumen 41 is incorporated within the stimulation lead to provide greater infusion selectivity. More specifically, Figure 14 shows that the second lumen 41 terminates at end 39 which is intermediate between the distal tip of the stimulation lead and the proximal end thereof. This lumen 41 communicates with the set of infusion ports 37 which are spaced from the end 39 of the lumen 41 towards the proximal end of the lead. The first or central lumen 24 then communicates with the infusion ports 35 that are located distally of the end 39 of the second lumen 41.

During treatment, it may be desirable to administer nutrients and/or medications to different parts of the disc being treated. Furthermore, it may be desirable to provide the nutrients/medications to these different locations within the disc at differing flow rates and at differing times and frequencies. With the provision of a dual set of lumens, a physician has the ability to selectively control infusion to two distinct areas within the disc, and can vary the treatment protocol between the two areas of the disc by selecting the particular dosing, frequency, and makeup of the infusion material to the distinct locations within the disc. This selective treatment capability may be advantageous where, for example, the distal end of the stimulation lead may be placed near the interface/transitional zone, and the tissue extending there along together with the annulus fibrosis may have particular needs in terms of the required type of nutrients and/or medication, while the tissue within the nucleus may have slightly different needs. Thus, the embodiment at Figure 14 provides the treating physician with additional options in providing effective treatment.

The particular sizes of the lumens, as well as the sizes and spacing of the openings 35 and 37 may be configured for optimal delivery of various types of infusion material. For example, assuming that the desired nutrient/medication to be delivered to the distal end of the stimulation lead was fairly viscous, it may be advantageous to provide the lumen 24 with a larger cross-sectional size, as well as to provide the infusion openings 35 of an increased size to accommodate the higher viscosity. As a further example, if the lumen 41 was to deliver a less viscous nutrient/medication, then the lumen 41 would preferably have a smaller cross-sectional area, and the openings 37 would preferably be smaller than the openings 35. Thus, one convenient way in which to control infusion is to advantageously arrange the particular size, number, and spacing of the infusion openings as well as the size of the lumens which deliver the infusion material through the openings.

It is further contemplated within the present invention to also provide non-uniform lumens, as well as infusion openings that vary in size within the same supplying lumen. As discussed above, the IDP 38 may be programmed for preset delivery of chemical "pulses". The IDP 38 is typically programmed to be in an "on" or "off" state to generate delivery of a set amount of fluid over a specific period of time. However, once the infusion material is released from the IDP, the IDP itself does not have control over the way in which the infusion material is dispersed through the stimulation lead. Assuming that a lumen of a stimulation lead has a uniform diameter with infusion openings also being of a uniform diameter, then the infusion ports located at the more proximal end of the device will most likely deliver a greater amount of material to the disc as opposed to the infusion ports located at the distal end of the device because there will be an inherent loss in the amount of fluid delivered downstream based on frictional losses within the lumen and the upstream openings communicating with the lumen. Therefore, in order to ensure equal distribution of infused material, it may be desirable to provide a lumen having a diameter that progressively enlarges as it extends towards the distal end of the device. Alternatively or in combination with the progressively changing lumen size, it may be desirable to provide infusion ports toward the proximal end of the device that are slightly smaller than the infusion ports located towards the distal end of the device to further help compensate for any frictional line losses.

Referring to Figure 15, yet another embodiment of the present invention is provided which further includes an inflatable portion 50 in the form of a bladder or balloon that is selectively inflated or deflated by an inflation line 52 extending conterminously with the central lumen. The inflatable portion is mounted to the exterior surface of the stimulation lead, and the inflation line 52 extends through an opening (not shown) in the sidewall of the lead that is covered by the inflatable portion 50. The inflation line 52 communicates with a source of compressed fluid (not shown), and the physician may inflate the inflatable portion 50 to a desired size. As also shown, the inflatable portion 50 is preferably placed along a location of the stimulation lead that does not cover or block any infusion ports 30, as well as any electrodes 22.

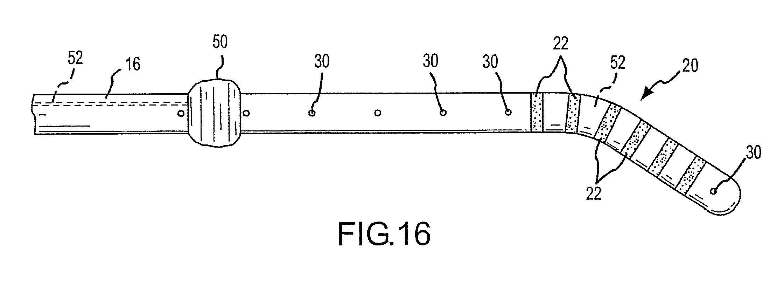

In some instances, the stimulation lead may reside within a patient for an extended period of time. As time passes, the stimulation lead may have a tendency to migrate or drift within the disc. Drifting of the stimulation lead can be problematic for a number of reasons, to include causing damage to the disc by penetration of the distal tip of the stimulation lead completely through the disc, as well as drifting of the stimulation lead so that it is no longer centered around/along the desired area of the disc to be treated. To maintain the stimulation lead in its desired position after the stimulation has been emplaced, the inflatable portion 50 may be inflated to the desired size, thereby serving as an anchor to help prevent drifting of the stimulation lead within the disc. In most instances, it is desirable to place the inflatable portion 50 near the distal tip of the stimulation lead to best prevent undesired drift of the stimulation lead; however, it is also contemplated within the present invention that the inflatable portion 50 may be selectively placed along other areas of the stimulation lead to best serve as an anchor. For example, as shown in Figure 16, the inflatable portion is located at the proximal end of the stimulation lead. Furthermore, it may be desirable to incorporate both a distally located inflation portion 50, and another inflation portion located at the proximal end of the device that would further help to prevent the stimulation lead from drifting or from being inadvertently removed.

Some disc tissue may have a tendency to adhere to a stimulation lead that has been emplaced within the disc for a long period of time, and/or the disc tissue may have a tendency to harden around the emplaced stimulation lead thereby making it more difficult to remove the stimulation lead. Thus, it is also contemplated within the present invention that the inflatable portion 50 could be provided to extend along a much greater distance of the stimulation lead, and the inflatable portion 50 could be inflated to a desired level prior to the stimulation lead being emplaced within a disc. When it is then desired to remove the stimulation lead, the inflatable portion could be deflated which would create a small gap or space between the surrounding disc tissue and the stimulation lead thereby easing removal of the stimulation lead.

Thus, the inflatable portion 50 can be used either as an anchor to maintain positioning of the stimulation lead within the disc, or the inflatable portion 50 can be used in a reverse role by enlarging the overall size of the stimulation lead once emplaced, but then reducing the overall size of the stimulation lead by deflating the inflatable portion when it is desired to remove the stimulation lead.

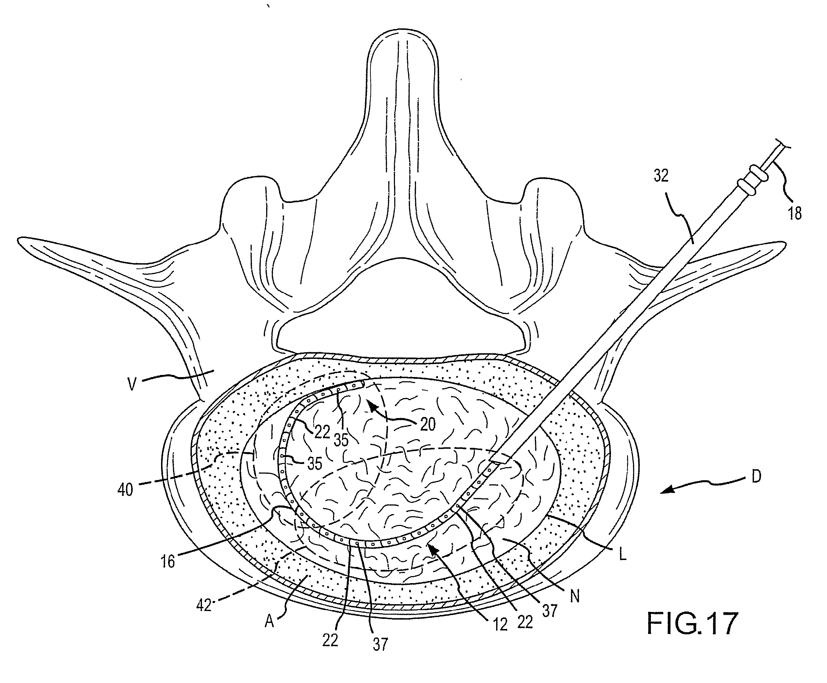

Referring to Figure 17, a stimulation lead is shown emplaced within a disc D, the stimulation lead generally corresponding to the embodiment shown in Figure 14. Two oval shaped areas 40 and 42 are shown surrounding the distal and proximal sections of the stimulation lead, respectively. These areas 40 and 42 may generally represent targeted treatment areas within the disc. In accordance with the embodiment of Figure 14, the physician has the option of applying different infusion materials through the separate sets of infusion ports 35 and 37 to specifically target the tissue located within the areas 40 and 42. Such treatment could be simultaneous, sequential, or any combination thereof. Furthermore, as mentioned above, selected sets of electrodes could be energized to provide treatment. For example, the electrodes may be wired so that the physician has the ability to energize two primary sets of electrodes, one set providing an electromagnetic field generated to cover area 40, and the other set providing an electromagnetic field to cover area 42. The electrodes may be wired and configured to provide generation of electromagnetic fields in any desired pattern along the length of the lead.

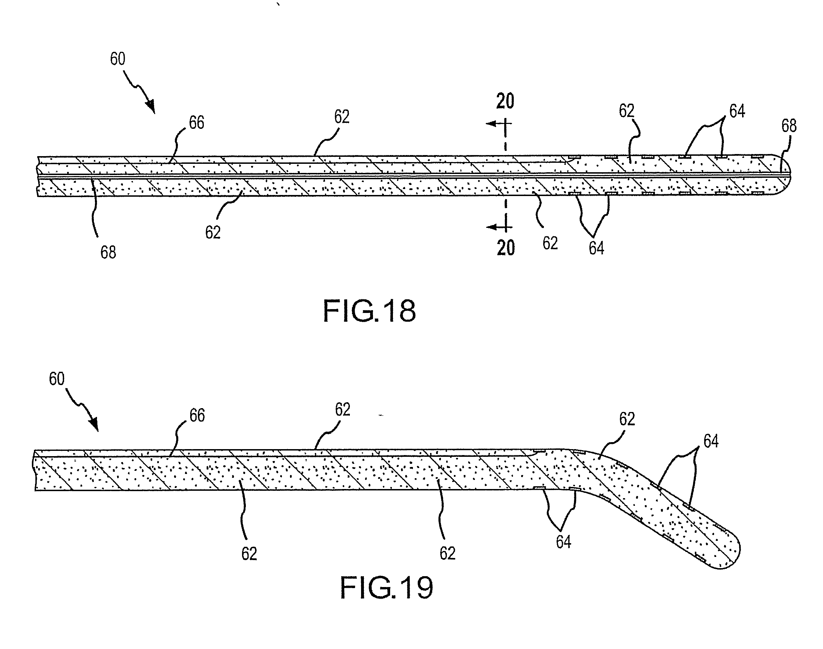

Referring now to Figures 18-20, yet another embodiment of the present invention is illustrated in the form of stimulation lead 60. For some treatments, it may be necessary to leave the stimulation lead emplaced within the invertebral disc for an extended period of time; however, for various reasons, it may not be possible to keep the stimulation lead emplaced for the amount of time to provide optimal treatment. In order to solve this particular problem, the embodiment of Figure 18 contemplates the use of various chemical agents/medications and nutrients incorporated within a dissolvable matrix that forms the body 62 of the stimulation lead 60. The electrodes 64 as well as the conductor(s) 66 could be formed with the dissolvable matrix in a molding process whereby a particular shape and size stimulation lead could be produced. The electrodes 64 could function the same as the electrodes 22 discussed above and could be produced in any desired pattern and wiring arrangement. The dissolvable matrix can be made of a material that is biomedically acceptable for enabling a time release of the chemical agents/medications and nutrients mixed within the matrix. The matrix is preferably a solid yet flexible material, allowing the stimulation lead to be steered with the use of an insertable stylet 56 which could be provided through the central lumen 68. However, it shall be understood that this central lumen 68 is optional, and the matrix may be manufactured of a material which is durable yet flexible enough allowing the practitioner to steer the stimulation lead without the use of a stylet. Accordingly, Figure 19 illustrates another embodiment wherein there is no lumen present, and a predetermined bend angle is formed in the stimulation lead enabling the lead to take the desired path through the disc when emplaced. Once inserted into the disc, the matrix would dissolve and the regenerating chemicals/medications and nutrients would slowly diffuse into the surrounding disc tissue leaving only the electrodes 64 and conducting wire(s) 66 to be removed at some later time.

With the embodiment shown in Figures 18 and 19, an infusion pump would not be required, and would thereby also allow for the subcutaneously placed pulse generator (IPG) to be significantly smaller. Similar to the combined pump/pulse generator device described above, this much smaller pulse generator could be rechargeable, or be powered by a battery source as desired.

In a modification to the embodiment of Figure 18, it is also contemplated within the scope of the present invention that a stimulation lead can simply comprise a dissolvable matrix having a desired combination of chemical agents/medications and nutrients, and no electrodes incorporated within the lead. In some cases, stimulation by an electromagnetic field may be unnecessary to achieve the desired regenerative and/or pain relieving disc response.

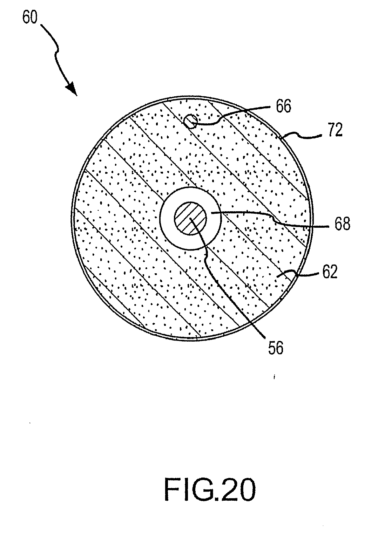

Figure 20 illustrates the designated cross-section of the device in Figure 18. Additionally, Figure 20 illustrates the use of an optional outer membrane 72 which could serve multiple purposes. One purpose for the membrane 72 would be to support the structural integrity of the matrix material of the body 62, thereby providing additional support for when the stimulation lead was emplaced. Additionally, this membrane 72 could serve as an osmotic membrane to help meter the rate at which the chemical agents/medications and nutrients were allowed to diffuse into the surrounding tissue. Thus, in addition to the matrix having a predetermined rate of diffusion, the membrane 72 could be used as an additional means to control the rate at which the chemical agents/medications and nutrients were delivered to the surrounding tissue. It is further contemplated that if the membrane 72 is provided only for structural support to the lead when emplaced, the membrane could be made of a material that quickly dissolves after being emplaced within the disc and the diffusion rate would be entirely controlled by the particular diffusion characteristics of the matrix.

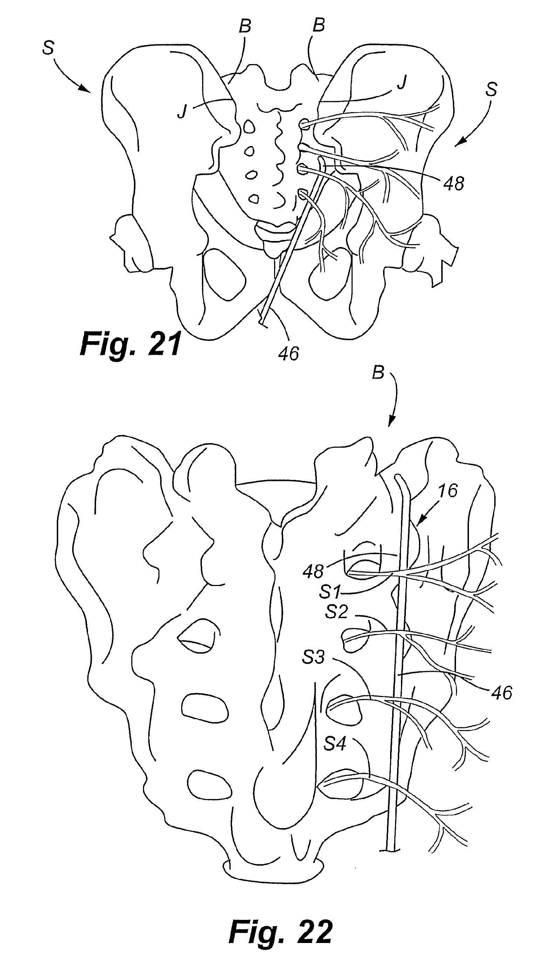

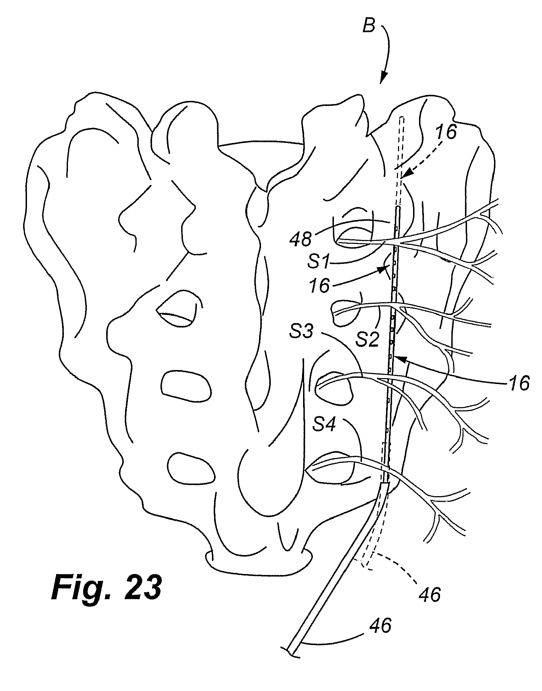

Referring now to Figure 21, in another aspect of the present invention, a stimulation device may be used to treat SI joint ailments. Figure 21 specifically illustrates a posterior view of the sacroiliac region with an introducer needle positioned for insertion along the sacroiliac region to a targeted area adjacent the SI joint J. Referring also to Figure 22, an enlarged posterior view of the sacrum bone B is shown wherein the introducer needle 46 has been fully inserted. In accordance with a method of the present invention for treatment of the SI joint, the introducer needle 46 is first inserted through the skin below and slightly medial to the inferior aspect to the SI joint and directed towards the inferior lateral edge of the sacrum. The introducer needle 46 is advanced to contact the dorsal aspect of the sacrum at the posterolateral edge. As shown, the needle 46 may have a slight curvature near the distal end thereof, shown as curve or bend 48, and the curvature of the bend 48 is then utilized to advance the needle lateral to the sacral foramen and medial to the dorsal aspect of the SI joint. The needle 46 remains in contact with the periosteum along the entire curvature of the sacrum. The needle tip ultimately advances to the superior edge of the sacrum lateral to the sacral foramen and medial to the SI joint. Appropriate positioning of the introducing needle is confirmed preferably both on Antero-posterior (AP) as well as lateral views. The stimulation lead 16 is then inserted through the introducer needle 46 until reaching the distal tip 48 of the introducer needle. The stimulation lead 16 is held in place by maintaining pressure on the lead. Referring now to Figure 23, the introducer needle 16 is withdrawn along a selected length of the stimulation lead 46 to expose the active number of electrodes 22 necessary to denervate the sacral nerve innervation to the SI joint. The dotted lines shown in Figure 23 for lead 16 represent the initial position of the lead after the needle 46 is withdrawn. After the lead 16 is exposed, local anesthetic and/or neurolytic agents and/or proliferant agents such as, but not limited to, phenol or alcohol, or Dextrose respectively could be injected through one or more of the infusion ports. The electrodes 22 may then be activated to ablate the surrounding neural tissue. The dotted lines for needle 46 in Figure 23 represent the position of the needle after it has been withdrawn and the lead is ready for activation. The solid lines in Figure 23 represent the next position of the lead 16 and needle 46 wherein both have been further withdrawn for purposes of conducting another activation to further denervate tissue, such as a circumstance when the initial ablation did not effectively cover the desired area of tissue.

With respect to the specific construction of the stimulation lead for use in a method of treating the SI joint, it may be constructed in the same manner as disclosed with respect to the prior description for treatment of a disc. More specifically, a stimulation lead may be selected having the most desirable arrangement of electrodes for the most optimal denervation of the targeted neural tissues.

The sacral nerves illustrated in Figure 23 include the lateral branches S1, S2, S3 and S4. In order to denervate each of the lateral branches, it may be required to sequentially apply energy to the stimulation lead as the introducer needle is repeatedly withdrawn along the path of insertion. Because of the variation of sacral anatomy, successful denervation may require two or more separate needle insertion angles in order to denervate the S1-S4 lateral branches. However, as discussed below with respect to the embodiments having multiple lead elements, it may be possible to avoid such multiple needle insertions. In addition to denervation of the sacral lateral branches, it may also be advantageous to denervate the L5 dorsal ramus as well as the L4 medial branch since there is some innervation to the SI joint from both of these additional nerve structures.

Although the figures show treatment along one side of the sacrum, it shall be understood that the same procedure may be repeated for treatment of the other side of the sacrum, by placement of the introducer needle in a symmetrical fashion on the corresponding opposite or contralateral side of the sacrum. In addition to electrical stimulation, it is also contemplated with respect to the method of treatment of the SI joint to also provide infusion in a combined electrical stimulation and chemical/drug infusion device. For example, infusion of collagen proliferants could be included in the method of treatment by use of a selected device including any one of the above-disclosed embodiments. Infusion of collagen proliferants such as dextrose, growth factors, and other nutrients may accelerate the healing process for some SI joint ailments. Depending upon the diagnosed ailment, infusion alone may be appropriate for the treatment, or in combination with some neural tissue ablation or stimulation. It is also contemplated in the method of the present invention to enhance neurolytic lesion size by infusion of substances such as Phenol, alcohol, and glycerin.

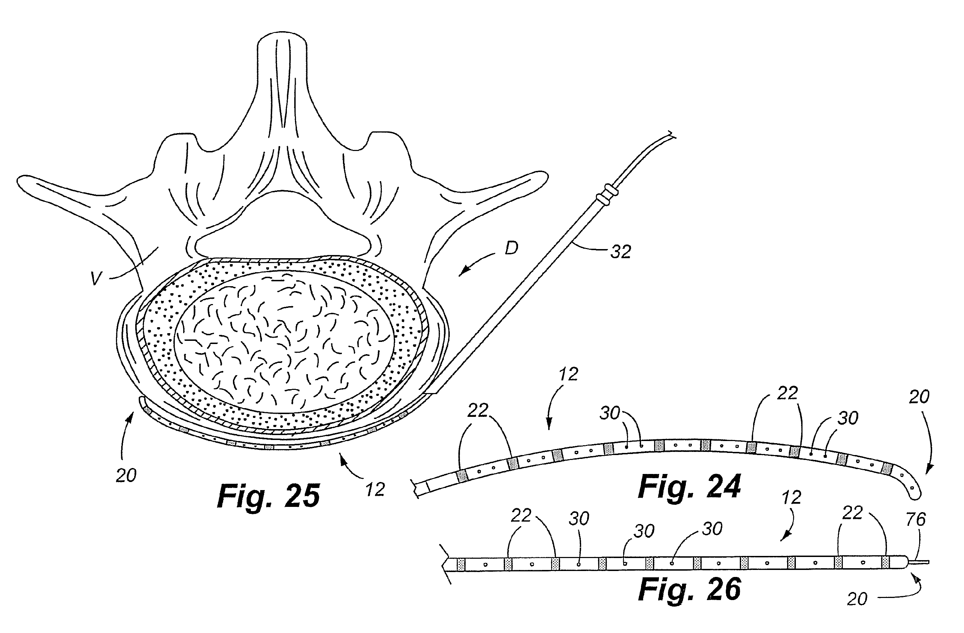

Figure 24 illustrates yet another preferred embodiment of the present invention. In this embodiment, the stimulation lead has a substantially uniform curvature over a selected length of the stimulation lead. The amount of curvature provides a desired angle for extending the stimulation lead within the targeted area of the body. Additionally, the distal end 20 of the stimulation lead is similar to what is shown in Figures 4 and 6, and therefore may have an additional bend that assists the medical practitioner in steering the stimulation lead once it has exited the distal end of the introducer needle 32. This particular shaped stimulation lead may also be advantageous in conducting treatment of the SI joint as discussed above with respect to Figures 21-23.

Figure 25 illustrates the stimulation device of Figure 24 for purposes of treatment of vertebral structures other than a disc, such as ventral vertebral structures that are to be ablated.

Figure 26 illustrates yet another preferred embodiment of the present invention wherein the stimulation lead is substantially liner or straight, and a stylet 76 is placed through a central lumen of the stimulation lead. For the embodiment of Figure 26, the stylet 76 may be used to steer the stimulation lead, and to provide the necessary stiffness to the stimulation lead during emplacement. Once the stimulation lead has been manipulated to the desired position, the stylet 76 can be removed while keeping the stimulation lead stationary. Then, the desired electrical stimulation procedure can be conducted along with any desired infusion.

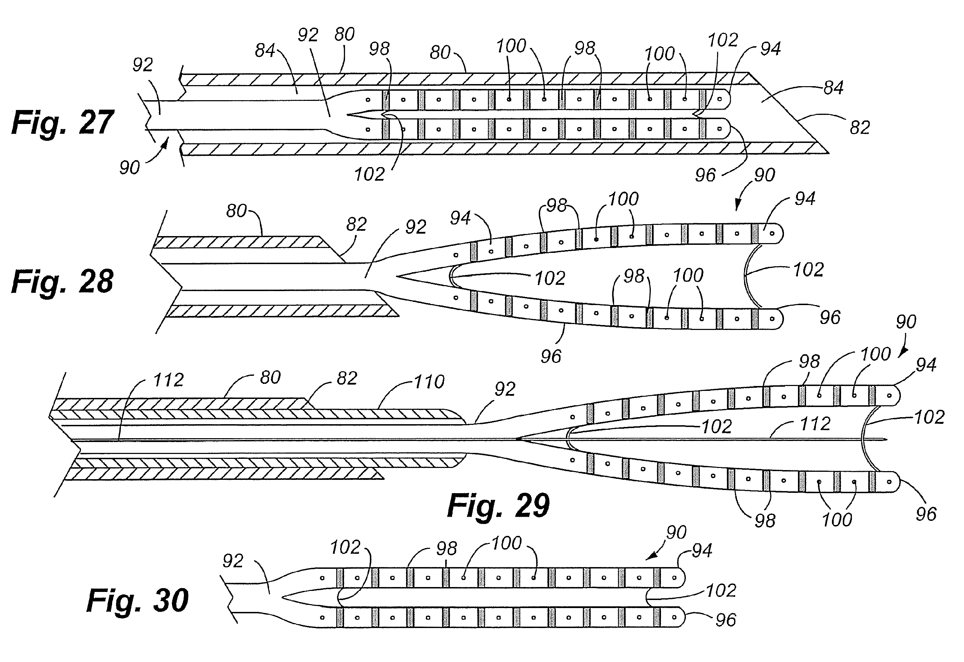

Figures 27 and 28 illustrate yet another preferred embodiment of the present invention. In this embodiment, an introducer needle 80 having a sharp distal tip 82 may be used to introduce the stimulation lead into the body. Once the introducer needle has been located, the stimulation lead 90 is then passed beyond the distal tip 82 of the introducer needle and placed on or near the targeted tissue. Alternatively, the introducer needle may be withdrawn and pressure maintained on the stimulation lead to keep the lead in the desired position. The stimulation lead in this embodiment has a pair of stimulation elements 94 and 96 that are deployed once the stimulation lead exits the distal end of the introducer needle. The stimulation elements 94 and 96 share a common base 92. As with the other embodiments, the stimulation lead 90 may also include a desired arrangement of electrodes 98 and infusion ports 100. One or more spring or deploying elements 102 are positioned between and attached to the respective stimulation elements. As shown in Figure 28, the deploying elements 102 can be in the form of curved springs, similar to leaf springs, which have enough spring force to spread or separate the stimulation elements of 94 and 96 away from one another, yet the springs can be collapsed to enable the stimulation lead to be placed back within the introducer needle or sheath. when the procedure is complete. Also, the spring elements 102 provide structural support to the stimulation elements in their deployed position to prevent excessive spread or displacement. Particularly in less dense tissue, it is advantageous for the stimulation elements to maintain their deployed configuration without further spread or displacement which might otherwise generate a burn lesion that is too large or a burn lesion that is not sufficiently concentrated. With respect to electrical stimulation, excessive spread or displacement of the leads may result in a weakened electrical field that does not adequately stimulate the targeted tissue. As shown, the stimulation elements are spread in a manner such that they extend radially beyond the diameter of the introducer needle. The extent to which the stimulation elements are spread or separated from one another can be dictated based on the desired spacing of the stimulation elements for the particular procedure being conducted. It should also be understood that only one spring element or more than two spring elements can be used between each pair of stimulation elements to optimize the desired configuration of the stimulation elements. Additionally, the particular type of material and the size of the springs 102 may be selected to match the intended purpose of the stimulation lead where it may be desired to deploy the stimulation elements in various configurations. The embodiment of Figure 28 is well suited for neural ablation wherein it may be desirable to ablate a relatively large area, thereby minimizing or eliminating the need to reintroduce the introducer needle along with multiple stimulation lead deployments.

With respect to the specific construction and material used for spring elements 102, it is contemplated that the spring elements can be made from either metallic or thermoplastic materials. The particular material chosen should have elastomeric/ resilient characteristics which allows the spring elements to expand or open when the stimulation elements are freed from the insertion needle, but also allows the spring elements to collapse without undue force when the stimulation lead is withdrawn and placed back within the introducer needle. As shown in Figure. 28, the particular spread of the stimulation elements allows the stimulation lead to cover a larger area than would be possible if just a single stimulation element were present.

Figure 29 illustrates the embodiment of Figure 28, but further adds a stylet 112 which can be used to guide the placement of the pair of stimulation elements. Figure. 29 also illustrates a sheath 110 that is placed within the introducer needle 80, and the stimulation lead is housed within the sheath. In accordance with a procedure for use of the embodiment of Figure 29, the introducer needle 80 is located first, and then the sheath 110 is extended beyond the distal end 82 of the introducer needle. The sheath is located, and then the stimulation lead 90 is extended beyond the distal end of the sheath 110. The sheath may be a very flexible material, and the use of the stylet 112 captured within the sheath adjacent the stimulation lead allows the stylet 112 to then precisely position the stimulation lead. Alternatively, it is also contemplated that a procedure could be conducted wherein the sheath is moved to the most distal position within the body, and then the stimulation lead 90 is held in place while the sheath 110 is then withdrawn back over the stimulation lead 90, thereby exposing the stimulation elements 94 and 96 and allowing them to deploy. Yet further in the alternative, a procedure can be conducted whereby the introducer needle is moved to the most distal position within the body, the needle is withdrawn while the sheath and stimulation lead are held in place, and then the sheath is withdrawn while the stimulation lead is held in place. As with the embodiment of Fig. 28, once the ablation or electrical stimulation is completed, the stimulation lead 90 is then withdrawn back into the sheath 110, the sheath 110 is withdrawn back into the needle 80, and then the needle is withdrawn from the body.

Figure 30 illustrates yet another preferred embodiment of the present invention wherein the pair of stimulation elements 94 and 96 are more closely spaced to one another and maintain a very parallel relationship with one another. The stimulation lead shown in Figure 30 is especially adapted for conducting electro stimulation along the spinal cord.

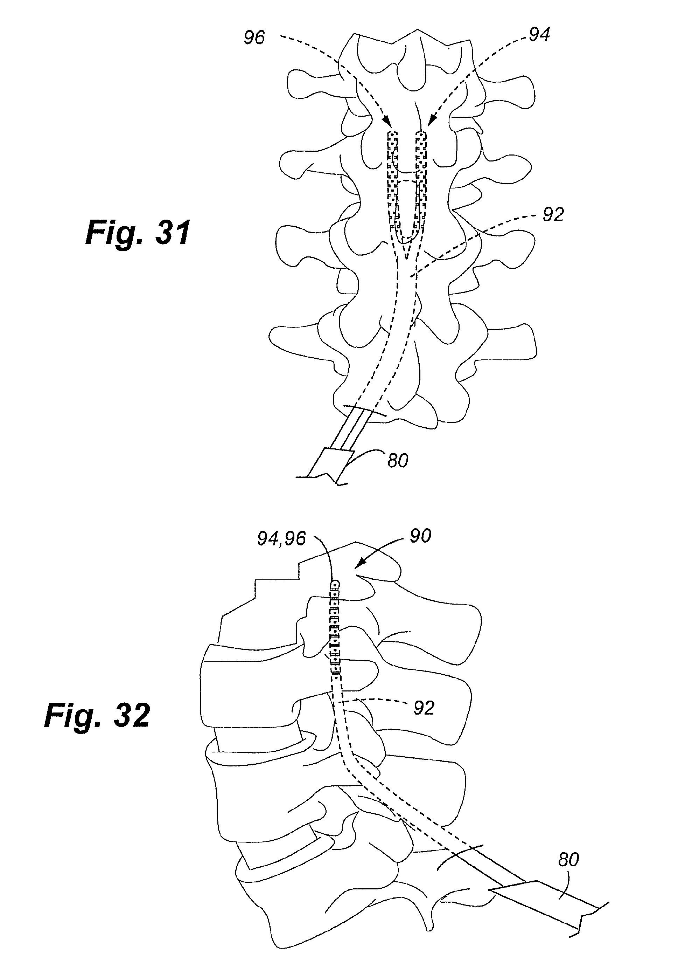

Figures 31 and 32 illustrate the stimulation lead of Figure 30 used in the procedure for electrical stimulation of the spinal cord and/or selected neural tissue adjacent the spinal cord. Although Figures 31 and 32 illustrate the stimulation lead for placement within the epidural space between vertebral structures, it shall be understood that the stimulation lead is well suited for treatment of other areas along the spine to include the ventral canal along the posterior longitudinal ligament, ventral dura, and the posterior aspect of the disc.

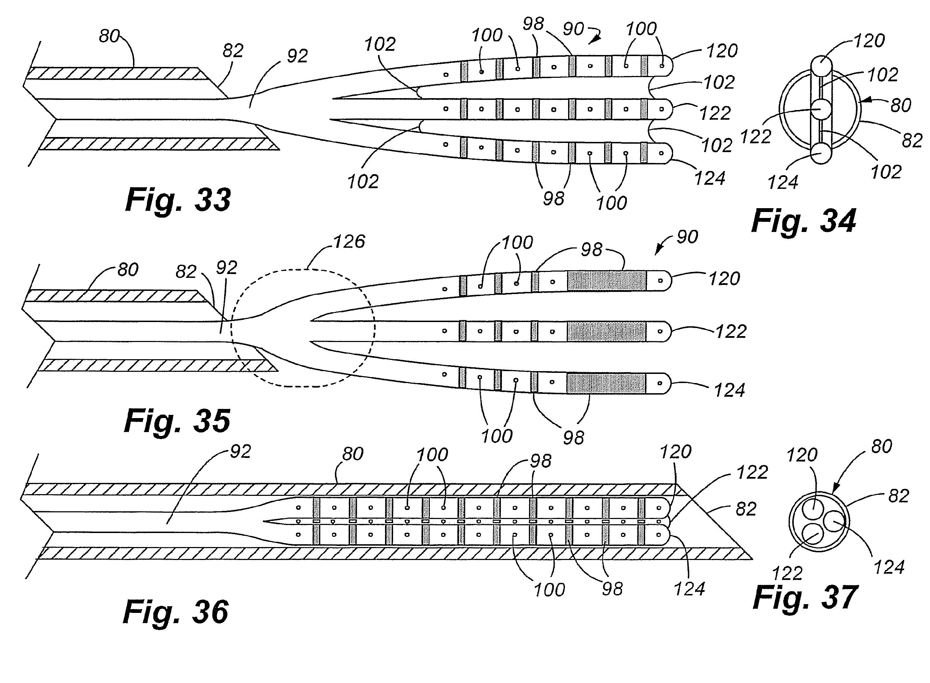

Figure 33 illustrates yet another embodiment of the present invention wherein the stimulation lead 90 includes 3 stimulation elements, 120, 122, and 124. The embodiment of Figure. 33 also illustrates a plurality of spring elements 102 placed between the stimulation elements. Figure 34 is an end view illustrating the linear spaced relationship between the stimulation elements 120, 122 and 124. For this particular embodiment, the provision of three stimulation elements may provide yet a larger area of tissue that may be ablated or otherwise treated, noting that the spread of the stimulation elements can cover yet a larger area depending upon the spread or gap between the stimulation elements.

Figure 35 illustrates yet another embodiment similar to the embodiment of Figure. 33, wherein three stimulation elements are present, but a different pattern of electrodes 98 and infusion ports 100 are provided. Additionally, the embodiment of Figure 35 does not require the incorporation of spring elements 102. Rather, the elastomeric/resilient nature of the stimulation lead material itself is enough for the stimulation elements to spread once they are freed from the distal end of the insertion needle. Highlighted area 126 on the base 92 of the stimulation device may be particularly elastomeric/resilient such that the stimulation elements 120, 122, and 124 spread in the depicted liner or flat orientation. Thus, the nature of the stimulation lead material can dictate the ultimate deployed orientation of the stimulation elements without the use of spring elements.

Figures 36 and 37 illustrate the embodiment of Figure 35 stored within the introducer needle. The stimulation elements 120, 122, and 124 are bundled together in order that the three stimulation elements may conveniently fit within the introducer needle. However, when the stimulation lead is deployed as shown in Figure 35, the elastomeric/resilient nature of the material at highlighted area 126 causes the stimulation elements to spread.

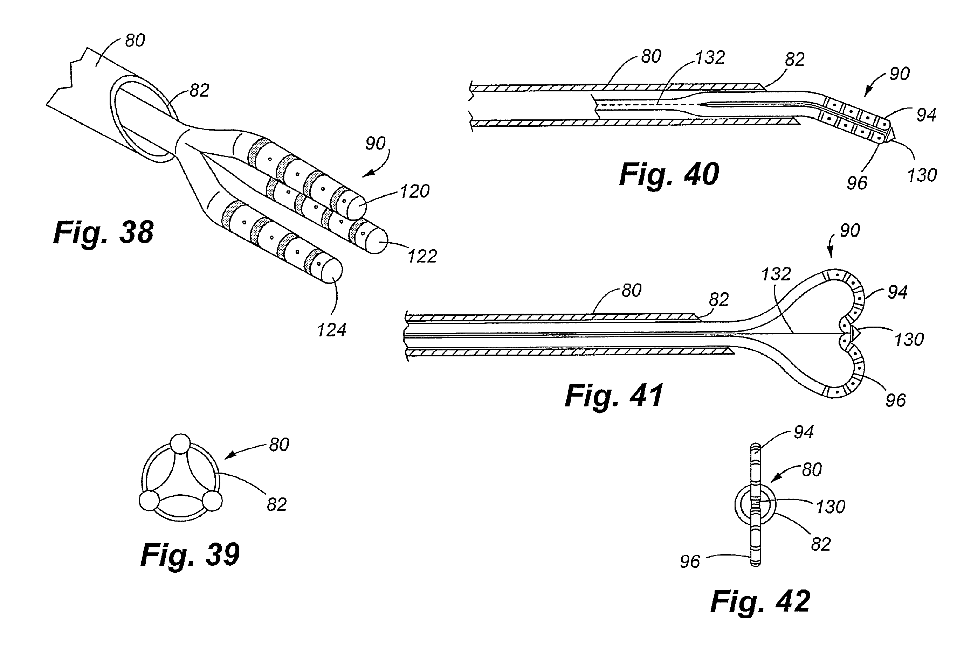

Figure 38 illustrates yet another embodiment of the present invention wherein three stimulation elements 120, 122, and 124 are provided, yet in their deployed position, the stimulation elements maintain a triangular configuration. Thus, this embodiment represents yet another arrangement of the stimulation elements that optimizes heat transfer or electrical stimulation of targeted tissue to be treated.

In order to stiffen or otherwise control any of the stimulation leads 90 shown in the embodiments of Figures 33, 35, and 38, a stylet (not shown) may also be used in the same manner as the stylet 112 shown and described with reference to Figure 29. Accordingly, a stylet captured within the needle or sheath adjacent the stimulation lead 90 allows the stylet to assist in precise placement of the stimulation lead.

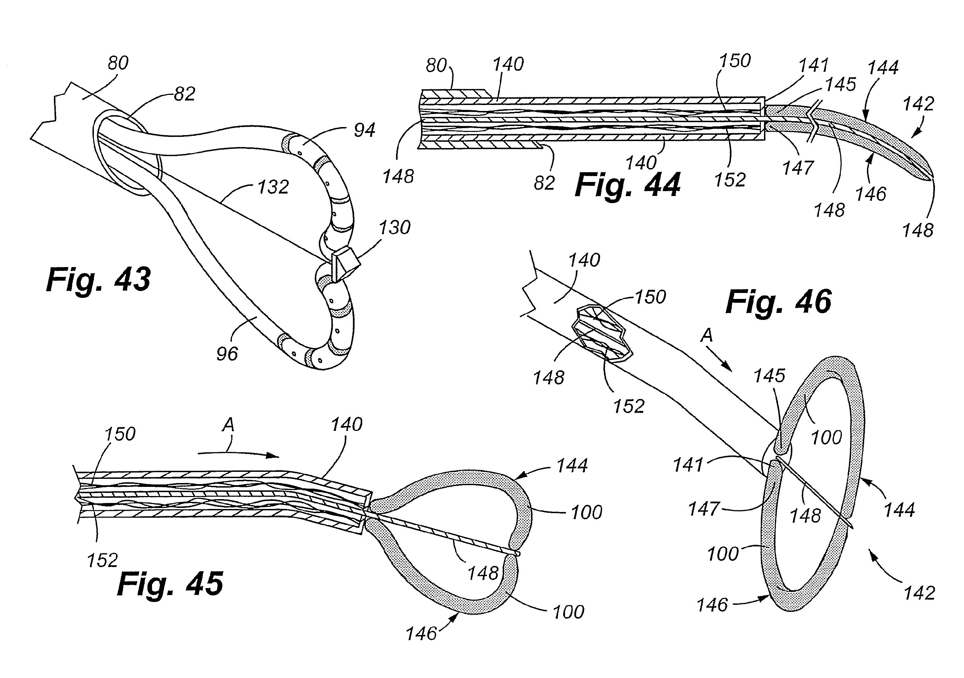

Figures 40-42 illustrate yet another preferred embodiment of the present invention wherein the stimulation device 90 includes a pair of stimulation elements 94 and 96 that are deployed by a different mechanism. The stimulation elements are deployed by use of a distal keeper or tab 130 that secures the distal ends of the stimulation elements. A tether or line 132 is attached to the tab 130. Once the stimulation elements are located, the tether 132 is withdrawn while applying force to the stimulation lead to prevent it from withdrawing with the tether. Accordingly, the stimulation elements spread apart from one another in the configuration shown. Optionally, the tab 130 may have a pointed distal tip that allows the stimulation lead to be better guided during emplacement. Figure 43 also illustrates the deployed position of the stimulation lead for the embodiment of Figures 40-42.

Figure 44 illustrates yet another preferred embodiment of the present invention. In this embodiment, a stimulation lead 142 is shown protruding from a sheath 140. The sheath is initially housed within an introducer needle 80. In the position shown in Figure 44, the introducer needle has been withdrawn or the sheath has been advanced beyond the needle thus exposing the stimulation lead 142. The stimulation lead 142 is not deployed from within the sheath and rather, the stimulation lead remains exposed. The stimulation lead in the embodiment of Figure 44 has two separate stimulation elements, namely, elements 144 and 146. These elements have respective proximal ends 145 and 147 that are secured to a distal cap 141 of the sheath. The stimulation elements 144 and 146 are preferably conductive along their entire length and therefore, form respective continuous electrodes. The stimulation elements 144 and 146 may be powered by control wires 150 and 152 that extend within the sheath 140 and proximally back to a stimulation source 14. Alternatively, the control wires can be eliminated and the sheath itself can be used as the conductor to provide power to the stimulation elements 144 and 146 since the proximal ends 145 and 147 in contact with the cap 141 can form continuous electrical pathways. However, if the sheath is used as the conductor, the outer surface is insulated to prevent the sheath from also being an active electrical element to prevent it from heating or electrically stimulating the surrounding tissue. If control wires 150 and 152 are used to provide power to the stimulation elements, then each may be separately controlled to provide the requisite electrical current to each element. A central stylet or needle 148 extends through a slot in the end cap 141 of the sheath and between the stimulation elements 144 and 146. The distal ends of the stimulation elements are attached to the distal tip of the stylet 148. Additionally, the stylet 148 may be an active stimulation element by being electrically powered either by a control wire or by the sheath in contact with the stylet. Referring now to Figures. 45 and 46, in order to deploy the stimulation elements in a desired configuration, the sheath 140 is moved in the distal direction as shown by directional arrow A while the stylet 148 is held stationary. As shown in Figure 45, as the sheath is advanced, the stimulation elements are separated from one another. Figure 46 shows the sheath further advanced such that the stimulation elements are fully deployed. Once the stimulation elements are in the desired configuration, the procedure can be conducted to heat or electrically stimulate the surrounding tissue. After the procedure, sheath 140 is withdrawn allowing the stimulation elements to collapse back on the stylet 148. The sheath may then be withdrawn back into the introducer needle. The embodiment of Figure 144 is especially adapted for use in neuro ablation procedures where it is desired to ablate a relatively large area. The heated tissue surrounding the stimulation elements and stylet will coalesce thus creating one burn lesion. Since the stimulation elements may be deployed at variable positions depending upon how far the sheath is advanced over the stylet, the particular lesion size, or size of the electrical field for electrical stimulation, can be selected by selective advancement of the sheath.

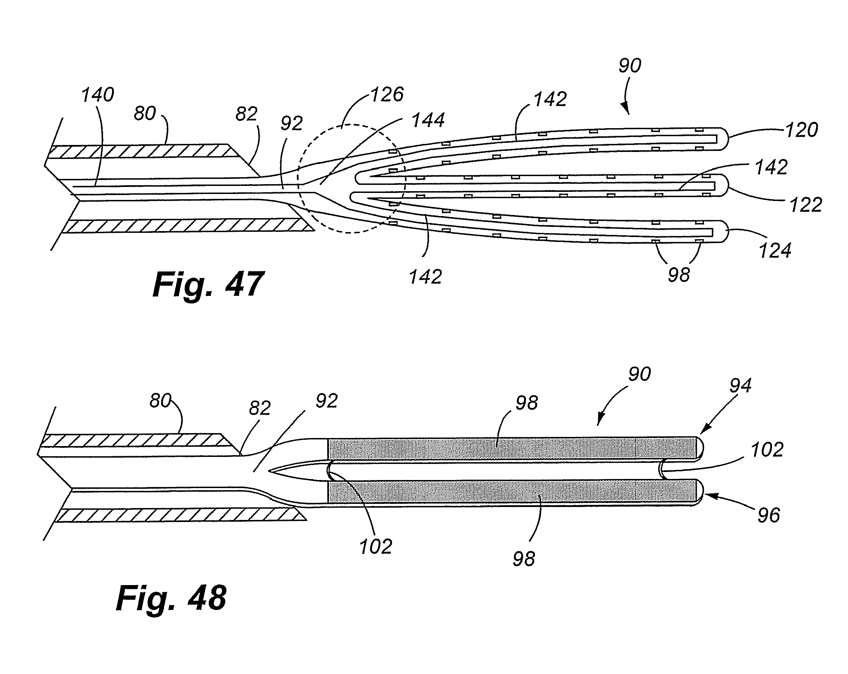

Figure 47 illustrates in cross section yet another embodiment of the present invention. In Figure 47, the stimulation lead is similar to the prior embodiments illustrated in Figures 30, 33 and 35, with the exception that a different mechanism is used to deploy the stimulation elements in the desired configuration. In Figure 47, a central lumen or opening 140 extends through the base 92 of the stimulation lead. The central lumen then separates into respective branches 142 that extend along a selected length of each of the stimulation elements 120, 122 and 124. The central lumen 140 may be enlarged in the area 126 thus forming a larger central chamber or opening 144. After the introducer needle 80 has been withdrawn to expose the stimulation elements, a fluid source is pumped into the central lumen 140 thereby pressurizing the central lumen 140 and each of its branches 142 with the pressurized fluid. The stimulation lead in this embodiment is made of a material that has some degree of flexibility, and may have some degree of elasticity such that pressurizing the central lumen and branches 142 causes some change in shape of the stimulation lead especially around the area 126. Of course in this embodiment, the respective branches 142 terminate within each of the stimulation elements to prevent the pressurized fluid from escaping. Therefore, the hydraulic force of the fluid that fills the central lumen 140 and its branches 142 expands the stimulation elements in the desired configuration. The particular shape of the reservoir 144, as well as the size and shape of the branches 142 will dictate the deployed configuration of the stimulation elements. Selective application of varying hydraulic pressures can be used to modify the shape of the stimulation lead and therefore to optimize the procedure to be conducted.

Figure 48 illustrates yet another embodiment of the present invention. In this embodiment, the stimulation lead 90 comprises a pair of stimulation elements 94 and 96, similar to those illustrated in Figures 28 and 29; however, the stimulation elements in this embodiment instead of having a tubular configuration are substantially planer or flat. Also in this embodiment, similar to the embodiment of Figure 44, the stimulation elements are continuous electrodes along the length of the stimulation elements as opposed to separately spaced electrodes. In some procedures, it may be preferable to have flat stimulation elements which can be placed in very narrow passages or openings formed in the tissue from the introducer needle or sheath, yet it is still desirable to have a plurality of stimulation elements that may be deployed in a desired configuration to increase the size or shape of the lesion created by ablation, or the size or shape of the electrical field to be applied to the targeted tissue. In order to stiffen the stimulation lead 90, a stylet (not shown) may also be used in the same manner as the stylet 112 shown and described with reference to Figure 29.

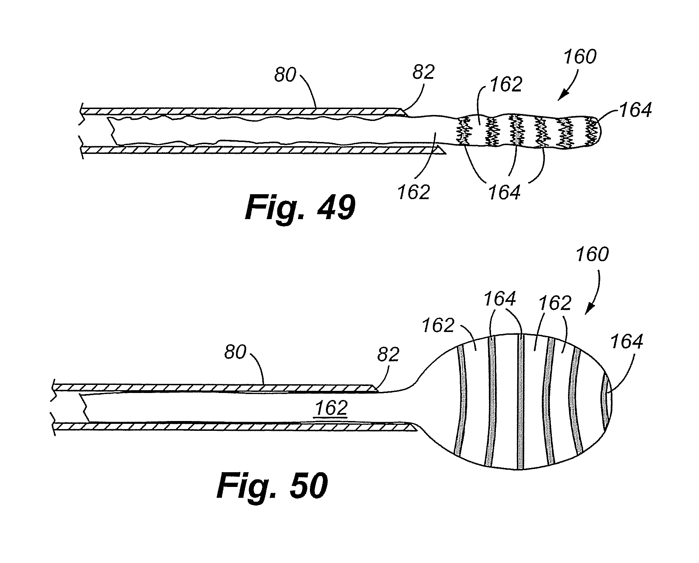

Figures 49 and 50 illustrate yet another preferred embodiment of the present invention wherein the stimulation lead 160 is made of a very flexible and elastomeric material, such as a synthetic rubber. The introducer needle 80 is preferably first advanced to the desired location where the procedure is to be conducted, and then the needle 80 is withdrawn thus exposing the stimulation lead 160 as shown in Figure 49. The stimulation lead 160 has a flexible and elastomeric body 162, and a plurality of electrodes 164 are secured to the outer surface of the body 162. The electrodes 164 may be electrical wires that have a zigzag appearance prior to the stimulation lead being inflated as shown in Figure 49. The electrodes 164 are not secured along their entire lengths to the body 162, but rather, at discrete points and there is some amount of slack in the electrodes between the points of connection which allows the electrodes to displace without breakage when the stimulation lead is inflated. In Figure 50, the stimulation lead is illustrated wherein a pressurized fluid source inflates the stimulation lead, and the amount of supplied fluid dictates the shape of the stimulation lead in its deployed position. As the stimulation lead is inflated, the body 162 increases in size and the electrodes 164 displace to a desired pattern. In the example of Figure 50, the electrodes 164 extend circumferentially around the body 162 in a plurality of bands that are spaced from one another. The interior of the stimulation lead may be hollow like a balloon, or the interior of the stimulation lead may have a lumen such that the stimulation lead has a much greater thickness. Additionally, the inflatable stimulation lead of this embodiment may have one or more stimulation elements provided in a configuration like the embodiment illustrated in Figures 38 and 47.

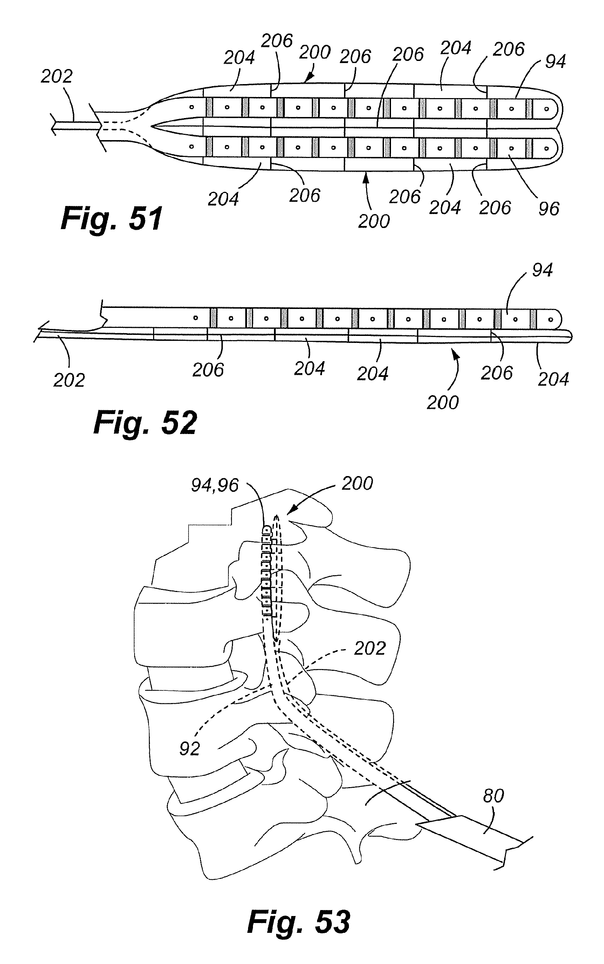

Figure 51 illustrates yet another preferred embodiment of the present invention including a stimulation lead that is similar to the one shown in the embodiment of Figure 30. In this embodiment, the stimulation lead therefore includes a pair of stimulation elements 94 and 96, with a plurality of electrodes spaced along the stimulation elements, as well as infusion ports. In addition to the stimulation elements 94 and 96, an inflatable balloon element 200 is provided in order to precisely position the stimulation lead during use. The balloon element may be adhered to one side of the stimulation elements. The balloon element is pressurized or depressurized by an inflation stem 202 that supplies pressurized fluid to the interior cavity of the inflatable balloon element. Inflating or deflating the balloon element a desired amount causes displacement of the stimulation elements 94 and 96 as discussed below. The balloon element 200 may be divided or separated into inflatable subelements 204 by dividers 206 that separate the interior cavity of the balloon element. A plurality of interior passageways (not shown) extend through the interior chamber and interconnect selected pockets 204 enabling only selected pockets to be inflated or deflated.

Figure 52 illustrates a side view of the balloon element attached to one of the stimulation elements 94, also illustrating the dividers 206 as well as pockets 204 that are formed by the dividers.

With respect to the balloon 200, it shall be understood that the stimulation lead and balloon may be placed within an introducer needle and then deployed through the introducer needle, similar to the manner in which the stimulation leads are deployed as shown in Figures 33 through 38. Accordingly, the distal tip of the introducer needle is advanced to a desired location, and then withdrawn, thus exposing the stimulation lead and balloon 200.

In yet another embodiment of the present invention, the balloon element 200 could represent a fixed paddle that helps to provide some rigidity or support to the stimulation elements 94 and 96. The fixed paddle would be flexible enough that it could fold-up within an introducer needle and when the stimulation elements were to be deployed, the fixed paddle helps to expand and support the stimulation elements at the location at which the targeted tissue is to be treated. Alternatively, a more fixed yet pliable paddle could be inserted using a surgical approach such as a luminotomy, luminectomy, etc.

Figure 53 illustrates the stimulation lead of Figure 51 and the balloon element 200 used in a procedure for electrical stimulation of the spinal cord and/or selected neural-tissue adjacent to the spinal cord. During such procedure, it may be advantageous to slightly shift or adjust the positioning of the stimulation elements such that the stimulation elements come closer to the spinal cord or neural-tissue to be treated. It is known that the cerebrospinal cord fluid (SCF) can interfere with targeted application of energy from the electrodes; therefore, the closer in proximity of the stimulation elements to the targeted tissue, the more effective the procedure. Once the stimulation lead has been emplaced, the balloon element may be inflated at selected pockets and at selected amounts in order to shift the positioning of the stimulation elements. The inflation or deflation of the balloon element will cause the balloon element to move its position and, therefore, provide a base against which the stimulation elements are also displaced for exact positioning. Additionally, the dorsal or exposed side of the balloon element that does not have the stimulation elements attached could have a roughened texture and/or barb-like elements incorporated therein to further stabilize the positioning of the stimulation elements and therefore decrease migration of the stimulation elements.

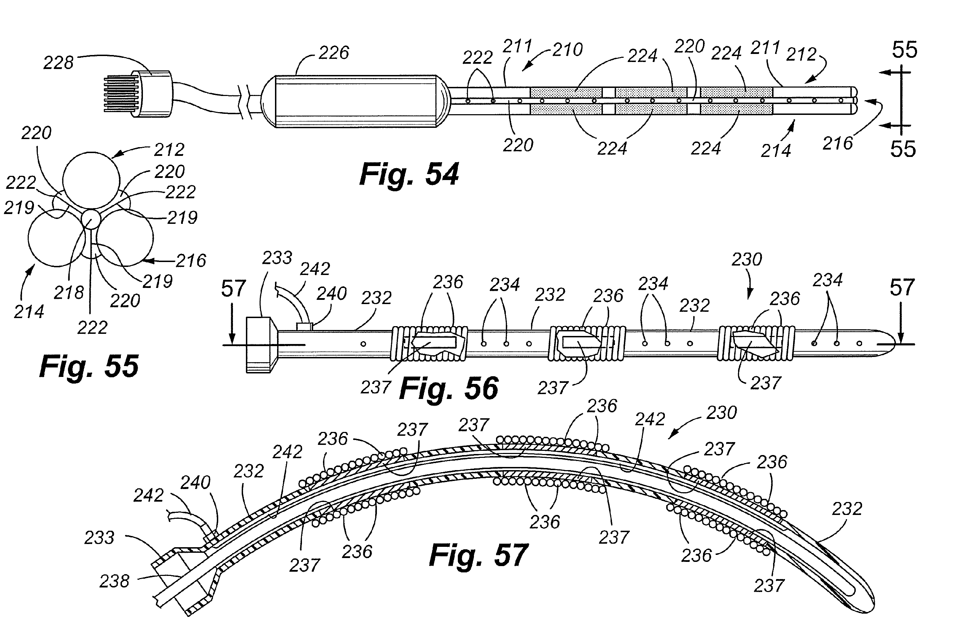

Figure 54 illustrates yet another stimulation lead 210 of the present invention including three stimulation elements 212, 214, and 216. Each of the stimulation elements includes a body 211, and a plurality of spaced electrodes 224, similar to the embodiment disclosed above in Figure 38. In the embodiment of Figure 54, a central infusion tube 218 is positioned inside the intersecting gap or space between the three stimulation elements. An insulating material 220 may surround the central infusion tube 218, thereby ensuring that each of the stimulation elements may be independently operated to deliver a desired energy to the targeted tissue, it being understood that the insulating material 220 prevents electrical contact between the respective stimulation elements. A plurality of infusion ports 222 may be formed through the insulation material and communicating with the central infusion tube 218 in order to selectively apply infusion material along the lengths of the stimulation elements. As shown in Figures 54 and 55, the infusion ports 222 are spaced along substantially the entire length of the stimulation elements; however, the infusion ports may be placed at only specific locations. Figure 54 also illustrates a handle 226 that can be configured to accept the proximal ends of the stimulation leads, and to house the bundled wires which may provide the electrical connection to the respective electrodes 224. It is also contemplated that each of the electrodes 224 may have its own temperature-sensing element, such as a thermocouple so that each of the electrodes 224 may be independently controlled to provide the desired electrical field or thermal energy. A multi-pin connector 228 is illustrated corresponding to the multiple wire conductors that may be used.

In the embodiment of Figures 54 and 55, with the use of three separate stimulation elements that each have three electrodes, nine independent active electrical conductive areas are provided. If each active service has a thermocouple, this results in an eighteen-conductor wire emerging from the handle.

With respect to the embodiment of Figure 54, it shall be understood that this stimulation lead can also be placed within an introducer needle such that when the introducer needle is withdrawn, the stimulation elements are exposed. Alternatively, the distal tips or ends of the stimulation elements 212, 214, and 216 may be sharp and stiff enough so that the stimulation lead may be placed without an introducer needle.

Figure 56 illustrates another stimulation lead 230 of the present invention. This stimulation lead 230 is characterized by a body 232 having a plurality of active elements/electrodes 236 comprising a tightly wound group of wires therefore forming a spring configuration. Preferably, the body 232 has a sidewall that is very thin. Therefore, the stimulation lead 230 is very flexible. A luer-lock 233 may be formed at the proximal end of the stimulation lead for connection to a handle and to a source of fluid that may be used to infuse targeted tissue through infusion ports 234. Referring also to Figure 57, the electrodes 236 make contact with electrical conductors 237 which may be either strips of electrically conductive material or cylindrical shaped bands of electrical material that are extruded or otherwise molded with the body 232. Power and control conductors may be housed in wire bundle 242 which extends through the body 232 and secured thereto by fitting 240. Wire bundle 242 may then traverse along the interior of the flexible body 232 to provide power to the conductors 236 that are in electrical contact with electrodes 237. An opturator 238 may be used to impart the desired bend or curvature when the stimulation lead is being emplaced. Thermocouples may be used at each conductor location. One great advantage with respect to the embodiment of Figures 56 and 57 is that this very flexible body along with the electrodes 236 which may easily twist or bend in the direction as dictated by the opturator 238 allows the medical practitioner to precisely place the electrodes 236 in very tight or hard to reach locations within the body.

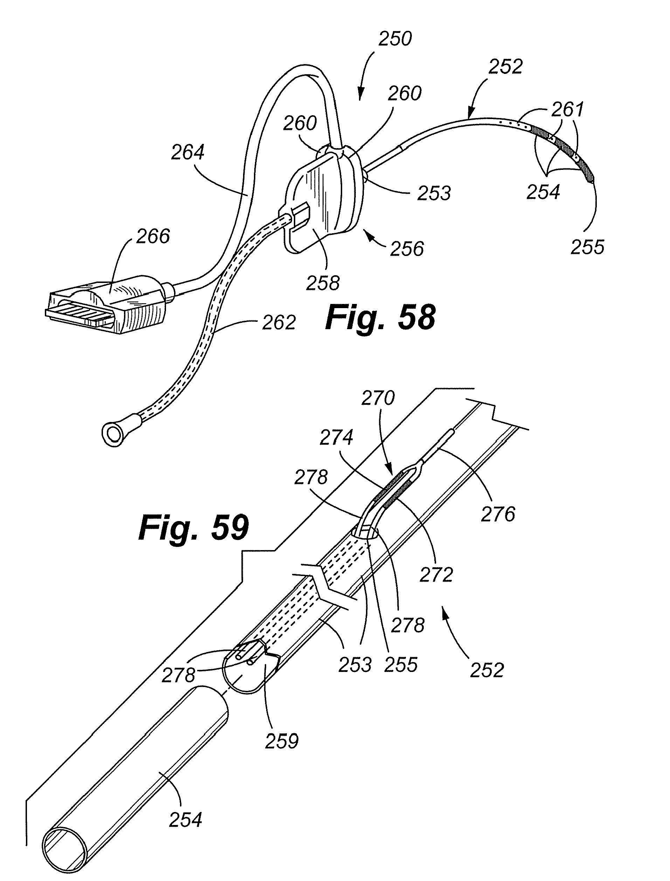

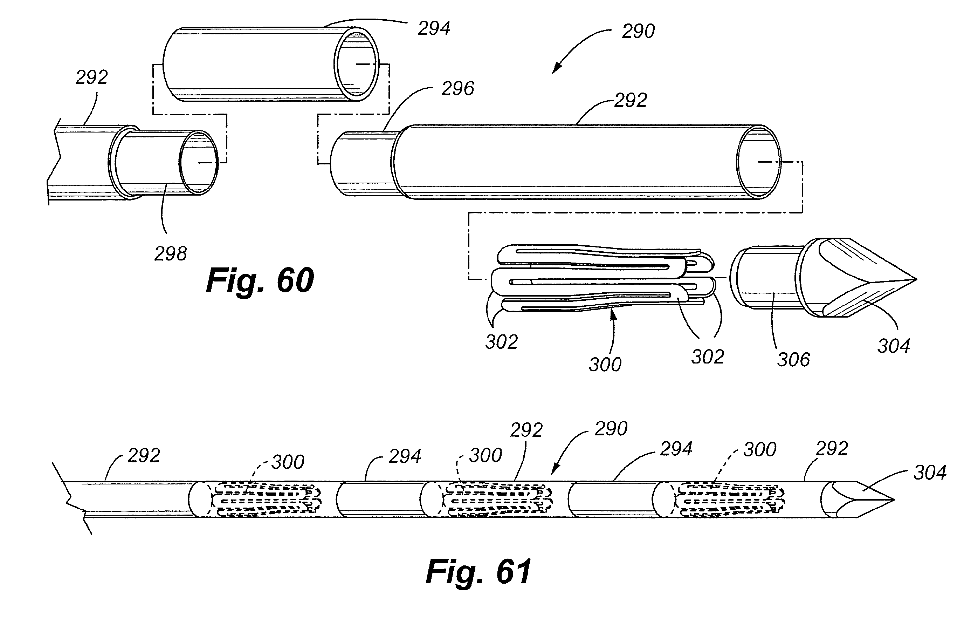

Now referring to Figures 58 and 59, a stimulation lead 250 is illustrated that can either be used as a reusable stimulation lead placed within an outer sheath (see e.g., Figure 60), or the stimulation lead 250 may be used by itself as a disposable stimulation lead. As shown, the stimulation lead 250 includes a stimulation body 252, and a plurality of electrodes 254 that are located at the distal end of the body 252. The tip 255 may be blunt, or may include a trocar type tip if the stimulation lead is to be forced through fairly dense tissue. A handle 256 is provided wherein the handle includes a central web 258, and a pair of transverse flanges 260 that extend substantially perpendicular to the central web 258. With the handle 256, the combination of the transverse flanges 260 and the web 258 allow the handle to be manipulated to rotate, twist, push, or pull the body 252 to precisely locate the stimulation lead. A fluid line 262 communicates with a central lumen 259 of the stimulation lead such that infusion may take place through selectively located infusion ports 261. A cable 264 and multi-pin connector 266 are provided to power the electrodes 254.

Referring specifically to Figure 59, an enlarged fragmentary perspective view is provided illustrating how the electrodes 254 may be secured to a non-conductive sheath 253. The non-conductive sheath and electrodes collectively make up the body. The non-conductive sheath 253 may be made of material such as plastic. An opening 255 may be made in the sheath to receive wires 278 which in turn are connected to conductors 272 and 274 forming a thermocouple. Junction 276 terminates the opposite ends of the pair of wires 278. The electrode 254 is in the form of a tubular member which fits over the sheath 253 and is secured to the sheath 253 by an appropriate adhesive, crimping, or other techniques. The wires 278 and thermocouple contact the electrode 254. The connection of the electrodes 254 over the sheath 253 is preferably watertight such that the central lumen 259 is shielded from the external environment. Wires 278 conduct power to the electrodes to include RF signals, as well as serving as conductors for measurement of electrical potential between the thermocouple elements 272 and 274.

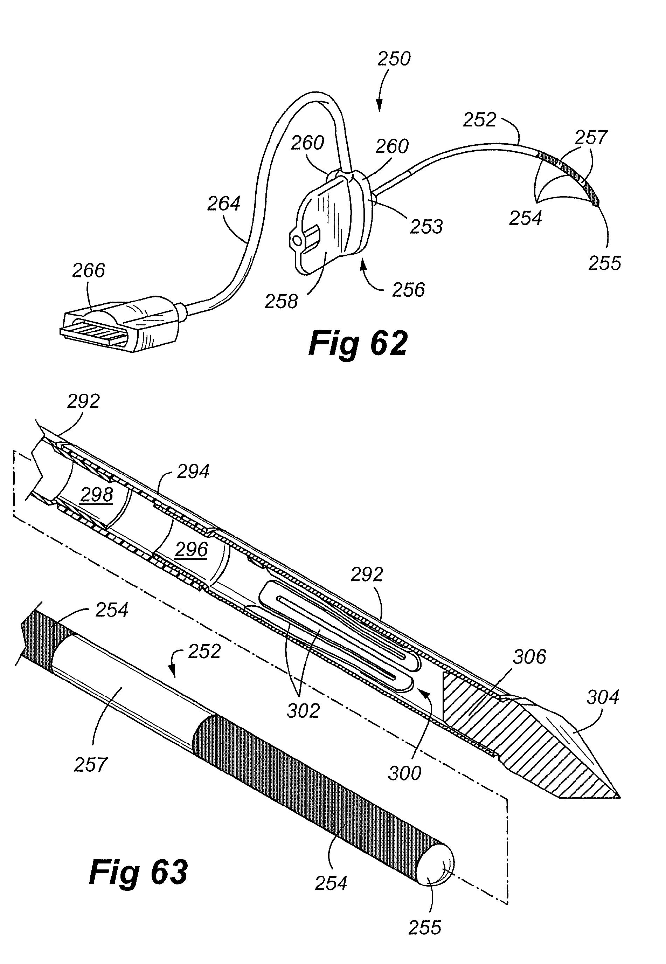

Figures 60 and 61 illustrate a disposable sheath 290 that can be used in conjunction with a reusable stimulation lead 250 of Figure 62. As shown in Figure 60, the disposable sheath 290 may include a plurality of conductive sections 292 which act as electrodes when placed in electrical contact with the electrodes of the reusable stimulation lead. Insulated non-conductive connectors 294 interconnect each of the electrodes 292. One simple method of connection is to provide smaller diameter flanges for the conductive sections, shown as flanges 296 and 298, and then press fit the sections together. Within each conductive section 292 is a spring finger conductor 300. The conductors are placed within each of the conductive sections 292 such that the traversing pattern of fingers 302 presses against the interior surface of the conductive sections 292. A desired shaped tip 304 may be provided for the sheath, shown in Figure 60 as a trocar type tip having a tapered sharpened end 304, and a base 306 that is received in the most distal end of the conductive section 292. Referring to Figure 62, the reusable stimulation lead 250 shown there is the same as stimulation lead 250 shown in Figure 8, except that the infusion line 262 has been eliminated. Referring to Figure 63, the body 252 of the stimulation lead 250 is inserted within the disposable sheath 290 so that the electrodes 254 of the stimulation lead align with the conductive sections 292, while the non-conductive sections 257 of the stimulation lead 250 align with the non-conductive sections 294 of the sheath. As the body 252 is placed within the sheath 290, the fingers 302 make frictional contact with the electrodes 254; the fingers 302 also being in contact with the conductive sections 292 creates an electrical pathway such that energizing selected one or all of the electrodes 254 results in energizing the corresponding conductive sections 292. With respect to measuring temperature at the conductive sections 292, temperature sensing elements such as a thermocouple may be incorporated in the stimulation lead 250 as disclosed above in Figure 59. Thus, the temperature of the conductive sections 292 may be measured since by conduction, thermal contact is maintained between the active areas of the stimulation lead and the conductive areas on the external sheath. One clear advantage of providing a disposable sheath 290 is that the sheath may be sized, shaped and otherwise designed for conducting a desired procedure. Use of a reusable stimulation lead lowers the cost of the procedure since the entire assembly does not have to be replaced in the next procedure; only the disposable sheath.

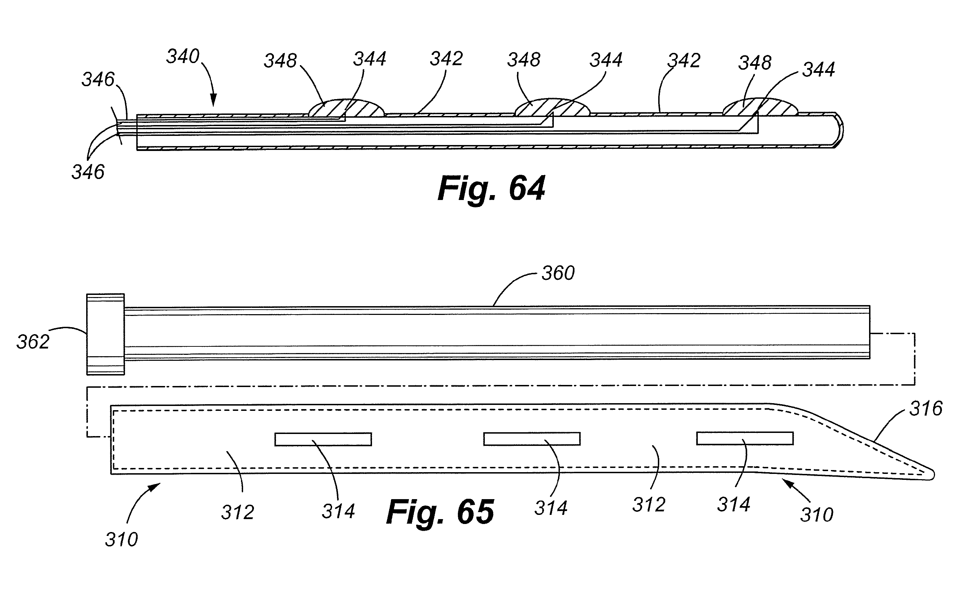

Referring to Figure 64, an alternate type of reusable stimulation lead 340 is illustrated. Figure 64 only illustrates the body 342 of the stimulation lead, it being understood that this embodiment may also include a handle, cable, an electrical connector, the same as shown in Figure 62. For the stimulation lead 340, a plurality of flexible electrical conductive pods 348 may be disposed at selected locations on the body 342. In the example of Figure 64, there are three linearly aligned conductive pods; however it should be understood that each of the pods 348 can be selectively placed such that they are spaced not only longitudinally along the length of the stimulation lead, but also circumferentially around the stimulation lead. Electrical energy is provided to each of the conductive pods 348 by pairs of wire conductors 346 that traverse through a central lumen of the body 342. Each pair of wires may include a thermocouple 344 that is placed in the electrical contact with conductive pods 348. As with the other embodiments, wire pairs 346 may be used to provide RF signals, as well as conductors for measuring differences of electrical potential at the thermocouples 344. The stimulation lead 340 may then be inserted within a disposable sheath, such as the one discussed above with respect to Figures 60 and 61, or with an alternate sheath assembly discussed below with respect to Figures 65-67.

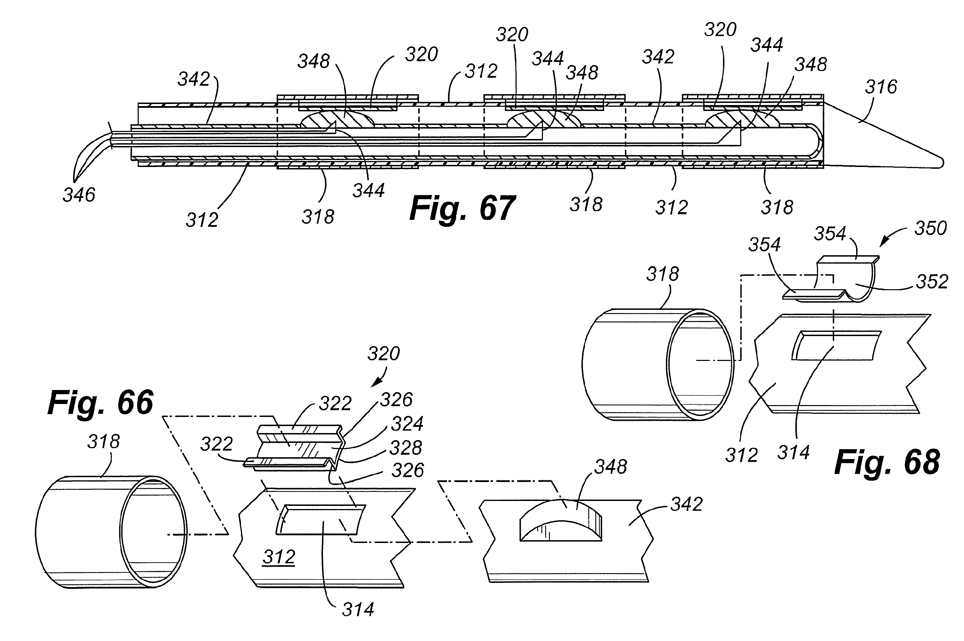

Referring to Figure 65, this alternative sheath embodiment 310 is characterized by a very flexible body 312 having a plurality of slots or openings 314 formed therein. A distal end of the body 312 includes a tip 316. The tip can be blunt or sharp, depending upon the intended use. Referring to Figure 66, electrodes 318 are cylindrical shaped sections that are slipped over the body 312, in the same manner as disclosed with respect to Figure 59. However, in the case of Figure 66, a bracket 320 is used to interconnect the electrodes from the reusable stimulation lead with the electrodes 318 formed on the sheath. As shown, the bracket 320 may include a pair of traverse flanges 322, sidewalls 326, and base 328. Accordingly, a channel 324 is formed between the sidewalls and base. The bracket 320 is placed in a corresponding slot 314 such that the flanges 322 rest on the outer surface of the body 312. When the electrode 318 is slipped over the body 312, the electrode 318 is aligned such that it covers the bracket 320. The electrode 318 is secured to the body 312 as by crimping, or by spot welding. Adhesive may also be used to ensure there is a liquid tight seal. In Figure 67, the thickness of the electrode 318 has been accentuated to enable understanding of how the electrode 318 is secured. However, it is preferable to provide a substantially smooth and continuous outer surface for the body 312 and electrodes 318, or at least a minimal protrusion of the electrode 318 above the outer surface of the body 312. One technique to ensure a smooth outer surface would be to form a channel in the body 312 to accept the electrode 318. When the reusable stimulation lead is placed within the central lumen of the sheath, the electrodes of the reusable stimulation lead make contact with the respective bases 328 of the brackets 320, thereby also energizing the respective electrodes 318. As shown in Figure 57, a flexible conductive pod 348 will make contact with the bracket 320. This arrangement is also shown in cross-section in Figure 66 where the three linearly aligned electrical conductive pods 348 make contact with the three linearly aligned brackets 320.

Referring back to Figure 65, an opturator 360 may be used when first emplacing the disposable sheath in a position where treatment is to be applied. Because of the very thin body 312, interior support of the opturator is necessary prior to insertion of the reusable stimulation lead. The opturator may have a standard end connection or flange 362 enabling it to be controlled in placing the disposable sheath 310.

Figure 68 illustrates another configuration of the embodiment of Figures 65-67 wherein an alternate shaped bracket 350 is provided. This bracket 350 includes a curved base 352, and a pair of opposing end flanges 354 which make contact with the outer surface of the body 312. The electrode 318 is slipped over the body 314, and the electrode 318 covers the bracket. The conductive pod 348 makes contact with the curved base when the stimulation lead 340 is placed within the sheath. Because of the curved shape of the bracket 350, some resiliency is present when the pod 348 makes contact thereby ensuring a good electrical connection.

One example medical procedure that may be conducted with one or more of the stimulation leads of the present invention includes treatment of the superior hypogastric plexis. For this procedure, the patient will be placed in a prone position. The vertebral body end plates of L5 and S 1 would be brought into alignment with a fluoroscopy beam. An introducer needle is inserted lateral to the superior articulur process of S 1 and infero-medial to the L5 nerve root and placed alongside or partially through the annulus of the L5-S1 intervertebral disc so that the curved tip is positioned at the inferior ventro-lateral aspect of the L5-S 1 disc as viewed on a lateral projection. A stimulation lead of the present invention is then inserted through the introducer needle and directed to lie at or slightly cephalad to the sacral prominons in the prevertebral space and extending across the width of the vertebral body as viewed on an AP projection. If the lead cannot be positioned all the way across the width as described, a bilateral approach can be employed. Myelogram safe contrast medium may be infused through the infusion ports of the lead to ensure appropriate tissue plane placement and away from unintended neural and/or vascular structures. Local anesthetic is then injected through the lead and a lesion or stimulation is carried out using determined protocols and activating the contacts necessary to achieve optimum therapy.

For each embodiment discussed above, it should be understood that each of the active electrical conductive areas or electrodes may be independently connected to a source of power such that each of the electrodes may be selectively energized or de-energized to provide the desired ablative pattern or electrical field. It is also desirable to provide a temperature-sensing element at each of the electrode locations, such as the illustrated thermocouples. Although thermocouples are shown, it shall be understood that other temperature elements may be used to sense or otherwise measure temperature such as RTDs, and others. With respect to control of each of the active electrical areas, it shall be understood that a controller can be used to measure temperature/energy applied at each of the conductive locations, as well as providing a visual indication as to how much energy has been applied over a period of time.

With respect to the distal tips of each of the different stimulation leads and disposable sheaths, it shall be understood that the distal tips may be active, electrical areas/electrodes. Thus, in addition to electrodes being selectively spaced along the length of the stimulation lead, the distal tips may also provide electrical or thermal energy to targeted tissue.

Based upon the foregoing, the present invention provides a combination electrical and chemical stimulation lead especially adapted for treatment of many types of ailments to include, disc ailments SI joint ailments, and other spine ailments to include treatment of structures that have large and diffuse innervations such as, but not limited to, the superior hypogastric plexus, sympathetic chain, ganglion impar, zygapophyseal joints, and others.

The various embodiments provide a treating physician with stimulation leads of various configurations, which optimizes a physician's ability to precisely position the stimulation lead, as well as to precisely direct both electrical and chemical stimulation.

While the above description and drawings disclose and illustrate embodiments of the present invention, it should be understood that the invention is not limited to these embodiments. Those skilled in the art may make other modifications and changes employing the principles of the present invention, particularly considering the foregoing teachings. Therefore, by the appended claims, the applicant intends to cover such modifications and other embodiments.

## Claims

1. An electrical treatment device comprising:
a stimulation lead having a pair of stimulation elements extending from a base forming a proximal end of said lead, said stimulation elements extending substantially parallel to one another and each stimulation element having a length;
at least one electrode integral with each stimulation element, said electrodes communicating with the source of electrical energy for providing ablative heat or electrical stimulation of tissue adjacent to said electrodes;
a balloon element secured to said stimulation lead, said balloon element extending along substantially the lengths of the stimulation elements, and said balloon element including one or more pockets that may be independently inflatable by supplying fluid through said inflatable balloon element.

2. An electrical treatment device comprising:
a stimulation lead having a plurality of stimulation elements, at least one electrode integral with each stimulation element, said electrodes communicating with a source of electrical energy for providing ablative heat or electrical stimulation of tissue adjacent to said electrode;
a central infusion tube extending between said plurality of stimulation elements and positioned at an intersection between said stimulation elements;
insulation material interconnecting said stimulation elements along a selected length of said stimulation elements;
a handle connected to said stimulation lead at a proximal end thereof.

3. An electrical treatment device comprising:
a stimulation lead having a body, and a plurality of electrodes connected to said body, said electrodes communicating with a source of electrical energy for providing ablative heat or electrical stimulation of tissue adjacent to said electrodes, said electrodes each comprising a conductor formed from flexible material extending around and in contact with an outer surface of said body;
a plurality of conductive elements integral with said body and disposed in contact with said electrodes, said electrical conductors being in electrical communication with an electrical source to provide electrical energy to said electrodes by conduction between said electrical conductors and said electrodes; and
a stylet placed through a central lumen of said body, said stylet being shaped to impart a desired shape onto said stimulation lead for steering said stimulation lead, said electrodes bending in response to the shape of the stylet and retained in position once placed, and said electrodes returning to their undeformed shape after removal of said stylet.

4. An electrical treatment device comprising:
a stimulation lead having a base forming a proximal end of said lead, said stimulation lead including at least one electrode formed on a body of said stimulation lead, said electrode communicating with a source of electrical energy for providing ablative heat or electrical stimulation of tissue adjacent said at least one electrode;
a handle connected to said proximal end of said stimulation lead;
a disposable sheath including a plurality of conductive sections and a plurality of non-conductive sections, two of said conductive sections being separated by an interconnecting non-conductive section;
a spring conductor placed in each of said conductive sections, said spring conductor contacting an interior surface of each corresponding conductive section; and
wherein said stimulation lead is placed in said disposable sheath, and said electrodes of said stimulation lead align with said conductive sections of said disposable sheath such that electrical energy from said electrodes are conducted through said spring conductors to said conductive sections.

5. A method of assembling an electrical stimulation lead, said method comprising the methods of:
(i) providing:
a stimulation lead having a base forming a proximal end of said lead, said stimulation lead including at least one electrode formed on a body of said stimulation lead, said electrode communicating with a source of electrical energy for providing ablative heat or electrical stimulation of tissue adjacent said at least one electrode;
a handle connected to said proximal end of said stimulation lead;
a source of electrical energy communicating with said at least one electrode;
a disposable sheath including a plurality of conductive sections and a plurality of non-conductive sections, two of said conductive sections being separated by an interconnecting non-conductive section;
a spring conductor placed in each of said conductive sections, said spring conductor contacting an interior surface of each corresponding conductive section;
(ii) placing said stimulation lead in said disposable sheath; and
(iii) aligning electrodes of said stimulation lead in contact with said conductive sections of said disposable sheath such that electrical energy from said stimulation leads are conducted through said spring conductors to said conductive sections thereby causing said conductive sections to act as electrodes in treatment of targeted tissue.

6. An electrical treatment device comprising:
a stimulation lead having a body, and at least one electrode integral with said body, said electrode communicating with a source of electrical energy for providing ablative heat or electrical stimulation of tissue adjacent said at least one electrode, wherein said electrode includes a flexible conductive pod formed on an outer surface of said body, and a thermocouple communicating with said conductive pod;
a disposable sheath having a body and a central lumen extending therethrough, said disposable sheath further including at least one slot formed through said body, a conductive bracket placed in said slot, and a conductive electrode placed over said body and in contact with said bracket; and
wherein said stimulation lead is placed in said central lumen of said disposable sheath, and said flexible conductive pod is aligned to make contact with said bracket within said central lumen thereby producing an electrical path from said conductive pod to said conductive electrode.

7. A device, as claimed in Claim 1, further including:
at least one temperature sensing element associated with said treatment device and disposed in electrical contact with a corresponding stimulation element.

8. A device, as claimed in Claim 2, further including:
at least one temperature sensing element associated with said treatment device and disposed in electrical contact with a corresponding stimulation element.

9. A device, as claimed in Claim 3, further including:
at least one temperature sensing element associated with said treatment device and disposed in electrical contact with a corresponding stimulation element.

10. A device, as claimed in Claim 4, further including:
at least one temperature sensing element associated with said treatment device and disposed in electrical contact with a corresponding stimulation element.

11. A device, as claimed in Claim 5, further including:
at least one temperature sensing element associated with said treatment device and disposed in electrical contact with a corresponding stimulation element.

12. A device, as claimed in Claim 6, further including:
at least one temperature sensing element associated with said treatment device and disposed in electrical contact with a corresponding stimulation element.

13. A device, as claimed in Claim 1, wherein:
said balloon element has a substantially planar configuration, and is in contact with said stimulation elements.

14. A device, as claimed in Claim 2, wherein:
said central infusion tube extends longitudinally along said plurality of stimulation elements, and infusion ports communicate with said central infusion tube and extending through said insulation material.

15. A device, as claimed in Claim 3, wherein:
said conductor includes a wire forming a helical spring shape extending around and in contact with the outer surface of the body.

16. A device, as claimed in Claim 4, wherein:
said spring conductor is resilient such that when said stimulation lead is placed in said disposable sheath, said spring conductor resiliently displaces in response to contact with the stimulation lead.

17. A device, as claimed in Claim 5, wherein:
said conductive bracket is cylindrically shaped.

18. A device, as claimed in Claim 6, wherein:
said conductive bracket is resiliently deformed in response to contact with said conductive pod.

19. In sub-combination, a disposable sheath for use in combination with an inserted electrical stimulation lead, said disposal sheath including:
a plurality of conductive sections and a plurality of non-conductive sections connected to one another in series, at least two of said conductive sections being separated by an inter-connecting non-conductive section:
said conductive sections and said non-conductive sections when assembled having a central lumen extending therethrough;
a spring conductor placed in each of said conductive sections, each of said spring conductors contacting an interior surface of each corresponding conductive section.

20. The sub-combination, as claimed in Claim 19, wherein:
each of said spring conductors includes a plurality of finger extensions, and said finger extensions being arranged in a side-by-side pattern.

21. The sub-combination, as claimed in Claim 19, further including:
at least one temperature sensing element incorporated in said disposal sheath and in electrical contact with a selected conductive section of said plurality of conductive sections.

22. The sub-combination, as claimed in Claim 19, further including:
a pointed tip connected to a distal end of said disposable sheath.

23. The sub-combination, as claimed in Claim 19, wherein:
at least one of said conductive sections includes a flange formed at a first end thereof, and an adjacent non-conductive section is secured to said at least one conductive section by contact of said flange with said non-conductive section.

24. A method of assembling a disposable sheath usable in combination with an electrical stimulation lead, said method comprising the steps of:
providing a plurality of conductive sections and a plurality of non-conductive sections;
securing the non-conductive sections to the conductive sections in series wherein at least two of said conductive sections are separated by an inter-connecting non-conductive section; and
wherein each of said conductive sections and non-conductive sections have a substantially cylindrical shape and respective opposite ends, wherein a first end of a conductive section is connected to an adjacent first end of a non-conductive section, and a second opposite end of said non-conductive section is connected to an end of another conductive section.

25. A method, as claimed in Claim 24, wherein:
said opposite ends of said conductive and non-conductive sections are joined to one another as by a press fit.

26. A method, as claimed in Claim 24, wherein:
at least one of said conductive and non-conductive sections includes a flange formed at an end thereof, and said flange making contact with an adjacent section to connect the sections to one another at the flange.

27. A method, as claimed in Claim 24, further including:
inserting a spring conductor in at least one of said conductive sections, said spring conductor contacting an interior surface of said conductive section.

28. A method, as claimed in Claim 24, further including the step of:
securing a pointed distal tip to a most distal section of said plurality of conductive and non-conductive sections.

29. An electrical stimulation device especially adapted for treatment of an Sl joint, said device comprising:
an introducer needle including a distal end, said distal end having a curved shape; a stimulation lead placed in said introducer needle and having an elongate, tubular configuration;
a plurality of electrodes positioned on said stimulation lead, said electrodes communicating with a source of electrical energy for providing ablative heat or electrical stimulation of tissue adjacent said electrodes.

30. A device as claimed in claim 29, wherein:
said stimulation lead has a diameter that progressively narrows from a proximal end of said lead towards said distal end thereof.

31. A device as claimed in claim 28 or 29, wherein said electrodes are arranged so as to extend either:
i) circumferentially around said stimulation lead, being spaced from one another longitudinally along a length of said stimulation lead; or
ii) substantially linearly along one side of said stimulation lead; or
iii) circumferentially around a portion of said distal end thereof.

32. An electrical stimulation device comprising:
an introducer needle including an open distal end;
a stimulation lead placed in said introducer needle and having an elongate, tubular configuration, said stimulation lead being curved along substantially a length thereof, said stimulation lead having a distal end with a bend incorporated thereon to assist in steering said stimulation lead; and
a plurality of electrodes positioned on said stimulation lead, said electrodes communicating with a source of electrical energy for providing ablative heat or electrical stimulation of tissue adjacent said electrodes.

33. An electrical stimulation device comprising:
an introducer needle including an open distal end;
a flexible stimulation lead placed in said introducer needle and having an elongate, straight, and tubular configuration, said lead having a central lumen extending therethrough;
a plurality of electrodes positioned on said stimulation lead, said electrodes communicating with a source of electrical energy for providing electrical stimulation of tissue adjacent said electrodes; and
a stylet placed through said central lumen of said stimulation lead, said stylet being shaped to impart a desired shape onto said stimulation lead for steering said stimulation lead.

34. An electrical stimulation device comprising:
an introducer needle including an open distal end;
a stimulation lead having a base forming a proximal end of said lead, said lead further having at least a pair of stimulation elements connected to said base and extending distally from said base, said lead being selectively placed between a stored position within said introducer needle and a deployed position wherein the base has at least a portion thereof remaining in said introducer needle and said stimulation elements extend distally beyond said introducer needle; and
at least one electrode integral with each stimulation element, said electrodes communicating with a source of electrical energy for providing ablative heat or electrical stimulation of tissue adjacent said electrodes.

35. A device as claimed in claim 34, wherein:
said stimulation elements are tubular shaped or flat shaped.

36. A device as claimed in claim 34 or 35, wherein:
said stimulation device further includes at least one spring element attached between said stimulation elements for selectively displacing said stimulation elements into a desired configuration when said stimulation lead is in the deployed position.

37. A device as claimed in claim 36, wherein:
said at least one spring element comprises a pair of spring elements, one spring element positioned between proximal ends of said stimulation elements and another spring element positioned between distal ends of said stimulation elements.

38. A device as claimed in claim 36 or 37, wherein:
said spring elements are configured as leaf springs, and are optionally made of metal or of a thermoplastic material.

39. A device as claimed in any one of claims 34 to 38, wherein:
said stimulation device further includes a stylet extending through said introducer needle.

40. A device as claimed in any one of claims 35 to 38, wherein:
said spring element expands to spread said stimulation elements when said stimulation lead is in the deployed position, and said spring element collapses when stimulation lead is placed in the stored position.

41. A device as claimed in any one of claims 34 to 40, wherein:
said stimulation lead is made of an elastomeric material and when said lead is placed in said deployed position, said stimulation elements spread apart from one another.

42. A device as claimed in any one of claims 34 to 41, wherein:
said at least a pair of stimulation elements include three stimulation elements.

43. A device as claimed in any one of claims 34 to 42, wherein:
when said lead is placed in the deployed position, said pair of stimulation elements spread apart from one another side by side and remain substantially planar with one another

44. A device as claimed in claim 42, wherein:
when said lead is placed in the deployed position, said three stimulation elements spread apart from one another into a triangular configuration.

45. A device as claimed in any one of claims 34 to 44, wherein:
said stimulation device further includes a tab connected to distal ends of the stimulation elements, and a tether extending through said introducer needle adjacent said stimulation element, said tether connected to said tab, and wherein when said stimulation lead is placed in the deployed position, said stimulation lead is held stationary and said tether is retracted causing said stimulation elements to spread apart.

46. A device, as claimed in any one of claims 34 to 45, wherein:
when said stimulation lead is placed in the deployed position, said stimulation leads spread apart from one another beyond a diameter of said introducer needle.

47. A device as claimed in claim 45, wherein:
said tab has a pointed tip.

48. A device as claimed in any one of claims 34 to 47, wherein:
said stimulation lead has a central lumen and a branch extending through each stimulation element, and fluid placed in said central lumen and said branches when said lead is placed in said deployed position, said fluid being under pressure to cause said stimulation leads to spread apart from one another.

49. An electrical stimulation device comprising:
an introducer needle including an open distal end;
a sheath placed in said introducer needle;
a stimulation lead having a base forming a proximal end of said lead, said lead further having at least a pair of stimulation elements connected to said base and extending distally from said base, said lead being selectively placed between a stored position within said sheath and a deployed position wherein the base has at least a portion thereof remaining in said sheath and said stimulation elements extend distally beyond said sheath and said introducer needle; and
at least one electrode integral with each stimulation element, said electrodes communicating with a source of electrical energy for providing ablative heat or electrical stimulation of tissue adjacent said electrodes.

50. An electrical stimulation device comprising:
a sheath having a central passageway and an end cap;
a stylet placed through said central passageway and through a slot formed in said distal end cap, said stylet extending beyond a distal end of said sheath;
a stimulation lead having a proximal end and a distal end, said proximal end of said lead connected to said end cap, and said distal end of said lead connected to said stylet, said lead having at least a pair of stimulation elements, said lead being selectively placed between a non-deployed position wherein the stimulation elements are collapsed against one another, and a deployed position wherein the stimulation elements are spread from one another by sliding said sheath in a distal direction along said stylet as said stylet is held stationary; and
at least one electrode integral with each stimulation element, said electrodes communicating with a source of electrical energy for providing ablative heat or electrical stimulation of tissue adjacent said electrodes.

51. A method of deploying an electrical stimulation device especially adapted for treatment of targeted tissue wherein deploying the stimulation lead optimizes ablative heat or orientation of an electric field with respect to the targeted tissue, said method comprising the steps of:
providing an electrical stimulation device comprising a stimulation lead having a base forming a proximal end of said lead, said lead further having at least a pair of stimulation elements connected to said base and extending distally from said base;
placing said lead in a stored position within at least one of a sheath and an introducer needle;
exposing said stimulation elements; and
deploying said stimulation elements by displacement of said stimulation elements in a configuration different from a configuration of said stimulation elements within said sheath or introducer needle.

52. A method as claimed in claim 51, wherein:
said exposing step includes moving said stimulation elements beyond a distal end of said sheath or said introducer needle.

53. A method as claimed in claim 51, wherein:
said exposing step includes withdrawing said sheath or said introducer needle to expose said stimulation elements.

54. A method as claimed in any one of claims 51 to 53, wherein:
said deploying step is achieved by said stimulation lead being made of an elastomeric material and exposing said stimulation elements causes them to displace.

55. A method as claimed in any one of claims 51 to 53, wherein:
said deploying step is achieved by at least one spring element attached between said stimulation elements, and said spring element being expanded as said stimulation elements are exposed.

56. A method, as claimed in any one of claims 51 to 53, wherein:
said deploying step is achieved by hydraulic pressure of a fluid that fills a lumen extending through said base and branches communicating with said lumen extending through said stimulation elements.

57. A method as claimed in any one of claims 51 to 53, wherein:
said deploying step is achieved by distal ends of said stimulation elements being connected to one another, and providing a tether secured to said distal ends and extending proximally through said at least one sheath or introducer needle, and wherein said stimulation lead is held stationary, and said tether is withdrawn in a proximal direction causing said stimulation elements to spread apart from one another.

58. A method as claimed in any one of claims 51 to 53, wherein:
said deploying step is achieved by provision of a stylet connected to distal ends of said stimulation elements, said stylet extending through said at least one sheath or introducer needle, proximal ends of said stimulation elements being secured to an end cap of said sheath, wherein said stylet is held stationary, and said sheath is advanced distally over said stylet thereby causing said stimulation elements to spread apart from one another.

59. A method as claimed in any one of claims 51 to 53, wherein:
said deploying step is achieved by hydraulic pressure of a fluid that fills an interior of said stimulation lead, and said stimulation lead expands in size when said fluid fills the interior.

60. An electrical stimulation device comprising:
a stimulation lead having a base forming a proximal end of said lead, said lead further having at least one stimulation element connected to said base and extending distally from said base, said lead being selectively placed between a stored position and a deployed position, said stimulation lead being made of a flexible and elastomeric material; and
at least one electrode integral said at least one stimulation element, said electrode communicating with a source of electrical energy for providing ablative heat or electrical stimulation of tissue adjacent said at least one electrode.
